# EUROPEAN PATENT SPECIFICATION

(11) **EP 1 737 949 B1**
(45) Date of publication and mention of the grant of the patent: **15.09.2010**
(21) Application number: 05718509.2
(22) Date of filing: 09.03.2005
(51) Int. Cl.: C12N 5/071, A61P 9/00, A61K 35/14, A61K 35/12

(54) **ENDOTHELIAL PROGENITOR CELLS AND METHODS OF USE THEREOF**
ENDOTHELVORLÄUFERZELLEN UND VERFAHREN ZUR VERWENDUNG DAVON
CELLULES ENDOTHELIALES PROGENITRICES ET LEURS METHODES D'UTILISATION

(30) Priority: 09.03.2004 US 551594 P
(43) Date of publication of application: 03.01.2007
(73) Proprietor: Absorber AB, 103 95 Stockholm (SE)
(72) Inventor: SUMITRAN-HOLGERSSON, Suchitra, S-141 33 Huddinge (SE)
(74) Representative: Aston, Heidi Frances
(86) International application number: PCT/IB2005/001072
(87) International publication number: WO 2005/085424

(56) References cited:
- REHMAN J ET AL: "Peripheral blood "endothelial progenitor cells" are derived from monocyte/macrophages and secrete angiogenic growth factors." CIRCULATION, vol. 107, no. 8, 4 March 2003 (2003-03-04), pages 1164-1169, XP008022340 ISSN: 0009-7322
- MUROHARA T ET AL: "Transplanted cord blood-derived endothelial precursor cells augment postnatal neovascularization." THE JOURNAL OF CLINICAL INVESTIGATION, vol. 105, no. 11, June 2000 (2000-06), pages 1527-1536, XP007900302 ISSN: 0021-9738
- PEICHEV M ET AL: "Expression of VEGFR-2 and AC133 by circulating human CD34(+) cells identifies a population of functional endothelial precursors." BLOOD, vol. 95, no. 3, 1 February 2000 (2000-02-01), pages 952-958, XP002251073 ISSN: 0006-4971
- NOWAK G ET AL: "Expression of vascular endothelial growth factor receptor-2 or Tie-2 on peripheral blood cells defines functionally competent cell populations capable of reendothelialization." CIRCULATION, vol. 110, no. 24, 14 December 2004 (2004-12-14), pages 3699-3707, XP007900295 ISSN: 1524-4539
- ELSHEIKH E ET AL: "Only a specific subset of human peripheral-blood monocytes has endothelial-like functional capacity." BLOOD, vol. 106, no. 7, 1 October 2005 (2005-10-01), pages 2347-2355, XP007900296 ISSN: 0006-4971
- ROMAGNANI P ET AL: "CD14+CD34low cells with stem cell phenotypic and functional features are the major source of circulating endothelial progenitors." CIRCULATION RESEARCH., vol. 97, no. 4, 19 August 2005 (2005-08-19), pages 314-322, XP002373137 ISSN: 1524-4571 -& "Online Data Supplements" CIRCULATION RESEARCH, [Online] pages 1-10, XP002373138 Retrieved from the Internet: URL:http://circres.ahajournals.org/cgi/con tent/full/01.RES.0000177670.72216.9b/DC1>

## Description

### BACKGROUND OF THE INVENTION

Angiogenesis is a process of new blood vessel development (neovascularization) from preexisting vasculature, while vasculogenesis refers to blood vessel formation from endothelial progenitors that differentiate *in situ*. Until recently, angiogenesis was considered the only means of adult neovascularization and vasculogenesis was thought to be limited to embryologic development. However, the existence of circulating endothelial progenitor cells (EPCs) has provided evidence that postnatal vasculogenesis also occurs in adults. The potential of EPC as therapeutic tools for rescue of tissues from ischemic damage has been suggested.

Thus, a need exists for a source of progenitor cells that can differentiate into endothelial cells.

### SUMMARY OF THE INVENTION

The invention is based on the discovery an endothelial progenitor cell. The progenitor cell is multipotent. The progenitor cell is capable of differentiating, e.g., in-vivo or in-vitro, into a endothelial cell or a smooth muscle cell. Accordingly, the invention features a endothelial progenitor cell culture, e.g., an *in-vitro* culture. The culture is an adhesion culture. Alternatively, the cells in the culture are in suspension. The cell is derived from hematopoietic tissue, such as blood, e.g. peripheral blood or bone marrow. The tissue is from a mammal such as human, a primate, mouse, rat, dog, cat, cow, horse, pig. The cell is immunoreactive for VEGFR-2 or Tie-2 and CD45 and non-immunoreactive with CD144. Optionally the VEGFR⁺ cell is also immunoreactive with CD14. The cells proliferate in vitro. The cells are capable of doubling 2, 3, 4, 5, 6,7, 8, 9 10, 11,12, 15, 20, 25 or more times and maintain there ability to differentiate into endothelial cells or smooth muscle cells.

The invention further features a method of transplanting the progenitor cell or the progenitor cell progeny in subject by providing VEGFR-2⁺ or Tie-2⁺, CD45⁺ and CD144⁻ progenitor cells or VEGFR-2⁺ CD14⁺, CD45⁺ and CD144⁻ progenitor cells and transplanting the cells into the subject. Transplantation confers a clinical benefit, e.g. alleviating one or more symptoms of the particular vascular disorder. Vascular disorders are diagnosed by a physician using methods know in the art.

An injured blood vessel is repopulated by administering to a subject a composition containing VEGFR-2⁺ or Tie-2⁺, CD45⁺ and CD144- progenitor cells or a composition containing VEGFR-2⁺ CD14⁺, CD45⁺ and CD144- progenitor cells. The composition is administered directly to the blood vessel (e.g., vein or artery), or arterial bed. Alternatively the composition is administered systemically.

A vascular graft is endothelized by contacting a vascular grafts with a composition containing VEGFR-2⁺ or Tie-2⁺, CD45⁺ and CD144⁻ progenitor cells or a composition containing VEGFR-2⁺ CD14⁺, CD45⁺ and CD144⁻ progenitor cells. The vascular graft is a vein. Alternatively the vascular graft is a artery. The graft is an artificial vascular graft, an allograft, or an isograft. The graft is contacted in vitro, in vivo, or ex vivo. The vascular graft is contacted prior to implantation in a subject. Alternatively, the graft is contacted with the composition after implantation in a subject.

By repopulating a blood vessel or endothelizing an vascular graft is meant that the blood vessel or graft has an increased amount of endothelial cells compared to blood vessel of graft that has not been treated with the progenitor cells. For example, the treated blood vessel or graft has an increased vessel diameter and media thickness as compared to an untreated blood vessel or graft.

The VEGFR-2⁺ or Tie-2⁺, CD45⁺ and CD144⁻ progenitor cells maintain the capacity to differentiated in vivo to endothelial cells or smooth muscle cells. Whereas the VEGFR-2⁺ CD14⁺, CD45⁺ and CD144⁻ progenitor cells maintain the capacity to differentiated in vivo to endothelial cells. The subject is for example a mammal such as human, a primate, mouse, rat, dog, cat, cow, horse, pig. The subject is suffering from a vascular disorder or vascular tissue damage. For example, the subject is suffering from Peripheral Artery Disease, Aneurysm, Renal (Kidney) Artery Disease, Raynaud's Phenomenon, Buerger's Disease, Peripheral Venous Disease, Varicose Veins, Blood Clots In the Veins. Transplantation and/or repopulation confers a clinical benefit, e.g. , alleviating one or more symptoms of the particular vascular disorder. Vascular disorders are diagnosed by a physician using methods know in the art.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In the case of conflict, the present specification, including definitions, will control. In addition, the materials, methods, and examples are illustrative only and not intended to be limiting.

Other features and advantages of the invention will be apparent from the following detailed description and claims.

### BRIEF DESCRIPTION OF THE DRAWINGS

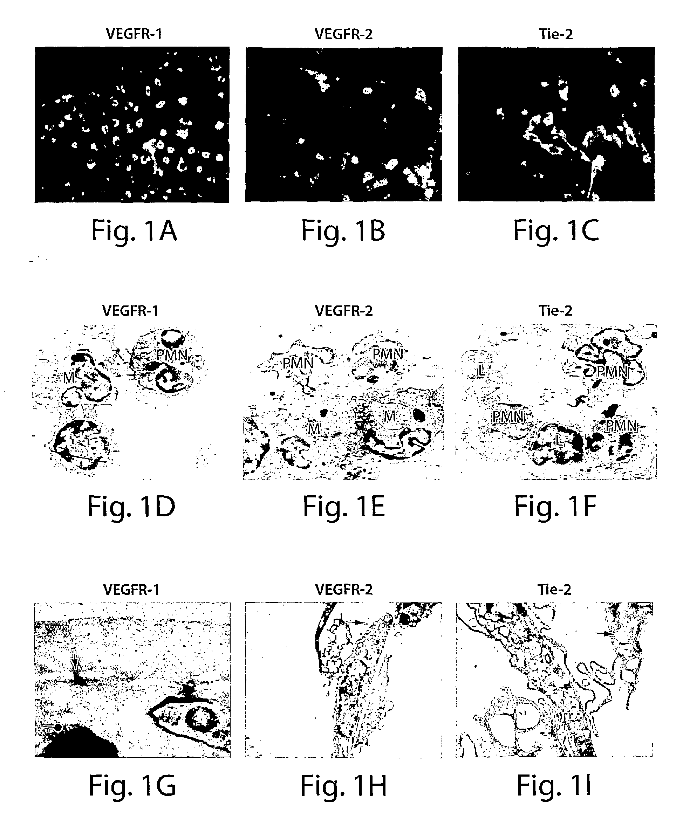

Figures 1 A-C are a series of Fluorescence micrographs (40x) illustrating that VEGFR-1+, VEGFR-2+ and Tie-2+ cells when stained with anti-VEGFR1, -VEGFR2 and Tie-2 antibodies were positive for their respective surface markers.
Figures 1 D-I are a series of electron micrographs illustrating that freshly sorted VEGFR-1+, VEGFR-2+ and Tie-2+ cell populations contained a mixture of cells comprising of mononuclear monocytes (M), macrophages (M), lymphocytes (L) and polymorphonuclear cells (PMN). G-I, TEM micrographs illustrate that VEGFR-2+ and Tie-2+ cells differentiated into endothelial cells demonstrating (G) the presence of weibel-palade bodies (rounded arrow) and tight junctions typical for endothelial cells (arrow) and (H, I) fenestrae (arrow).

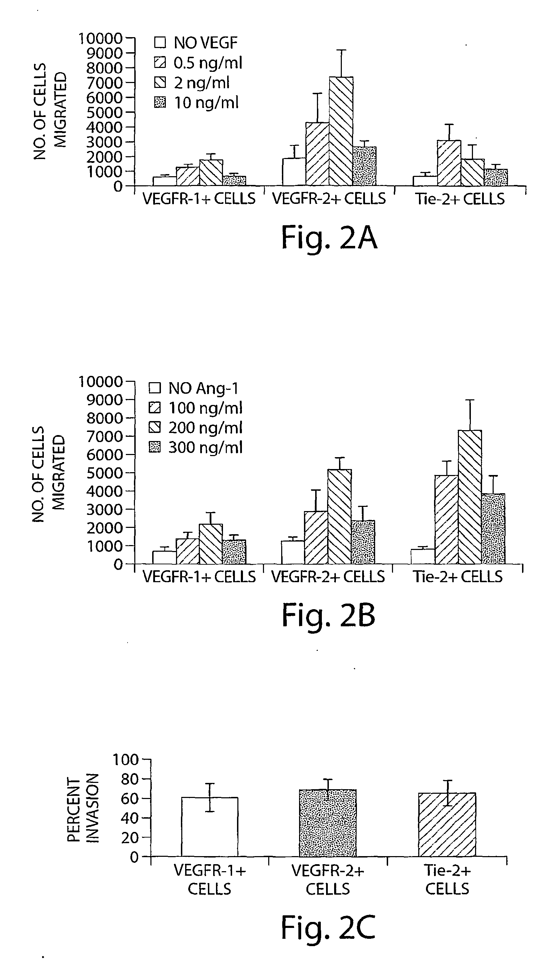

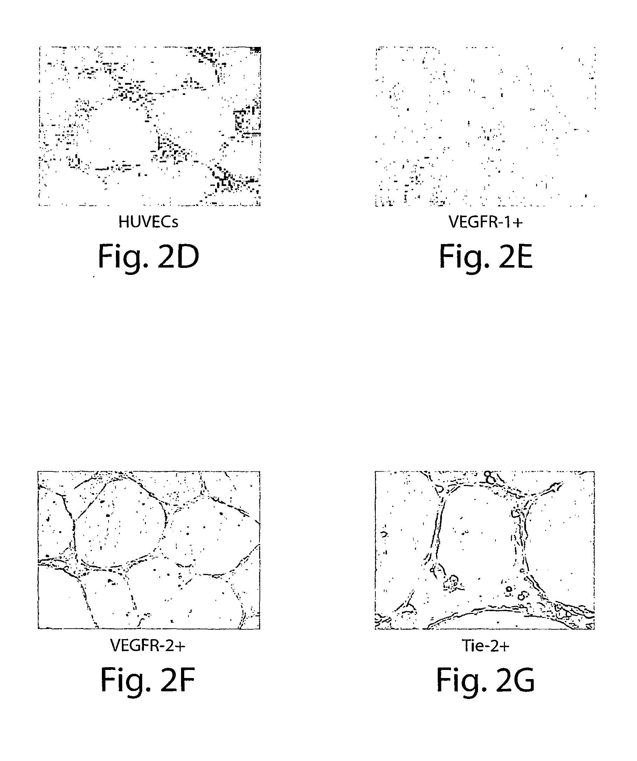

Figure 2 A-C are a series of bar charts showing that VEGFR-2+ and Tie-2+ but not VEGFR-1+ cells migrated towards VEGF and ang-1 and that all three populations of cells showed invasive capacity in a matrigel cell invasive assay. Results are presented as ±SEM.
Figures 2 D-G are a series of photographs showing that VEGFR-2+ and Tie-2+ but not VEGFR-1+cells formed capillary like tubule formation in matrigel. HUVEC were used as control cells.

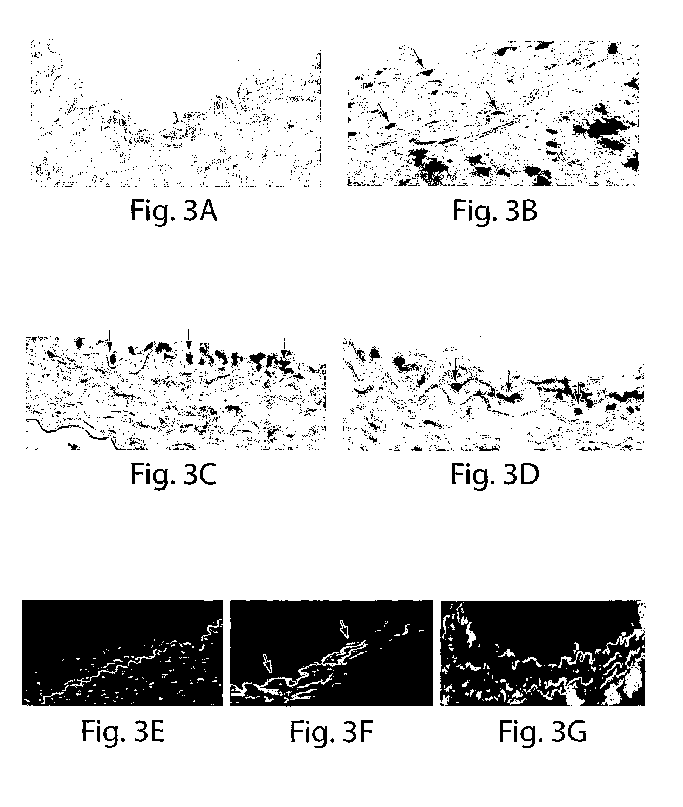

Figure 3 is a series of photographs. A&B, The human-nuclei antibody stained cells (black) in the human, but not control mouse artery. Immunohistochemical staining of sections from balloon-injured mouse femoral artery show the localization of transplanted human in the luminal surface of the seeded artery (C) VEGFR-2+ cells (arrows) at 4 weeks and (D) Tie-2+ cells (arrows) at 2 weeks. Original magnification 60x. Immunofluorescence staining of E, normal human artery, F, injured mouse artery transplanted with human Tie-2+ cells and G, sham transplanted mouse artery with the anti-human nuclei antibody (arrows).

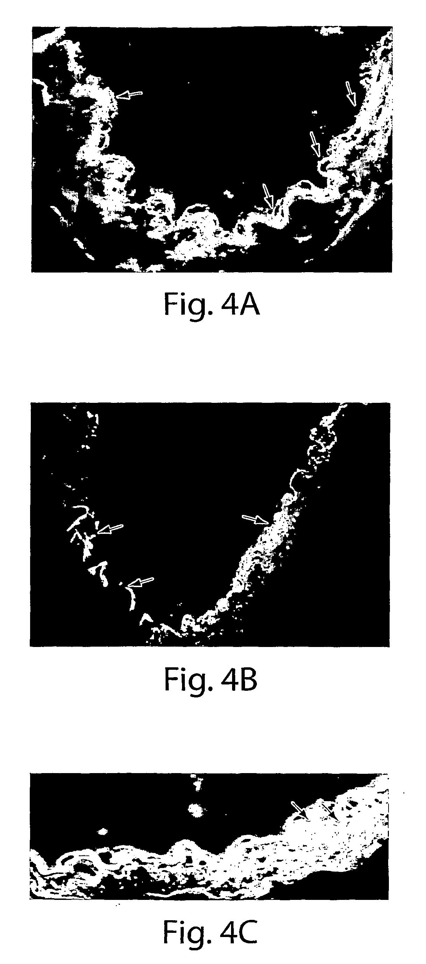

Figure 4 is a series of photographs showing that the EC phenotype of transplanted human cells was confirmed by double staining with human-nuclei antibody and antibodies to A, von Willebrand factor for VEGFR-2+ cells (double positive cells are stained yellow, arrows) and B, VE-Cadherin for Tie-2+ cells. When labeled with an antibody to α-actin, cells (green, filamentous staining pattern) in the media stained positive indicating the presence of smooth muscle cells. Original magnification 60x.

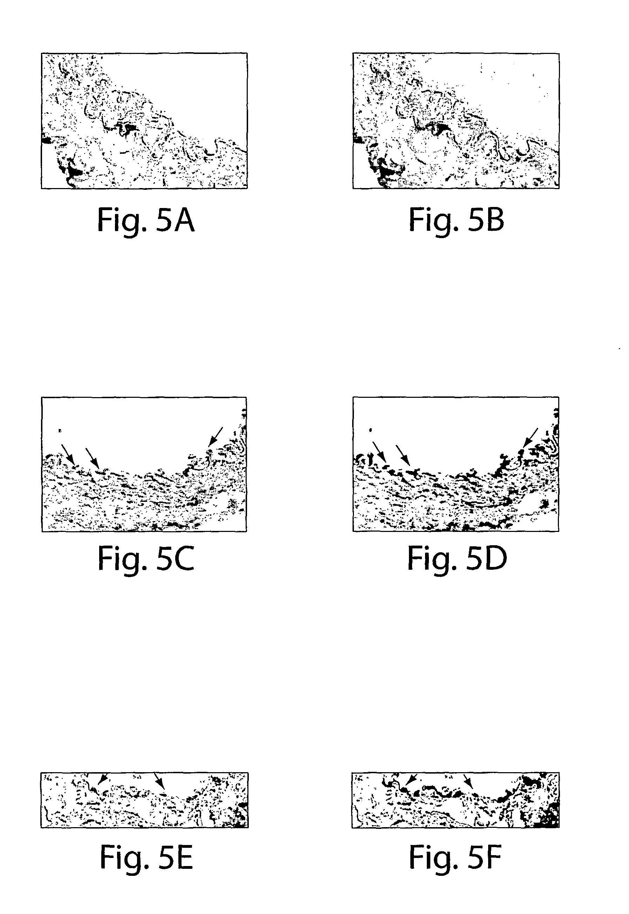

Figures 5 A-F are a series of photographs showing the analysis of tissue at 7 days showed extensive BrdU incorporation in VEGFR-2+ and Tie-2+ transplanted human cells *in vivo*. Representative consecutive sections A&B, of sham-transplanted mouse artery, and C-F, of transplanted mouse artery stained with anti-BrdU (brown cells) and the human nuclei antibody (black) showing proliferation in the transplanted VEGFR-2+ and Tie-2+ cells respectively.

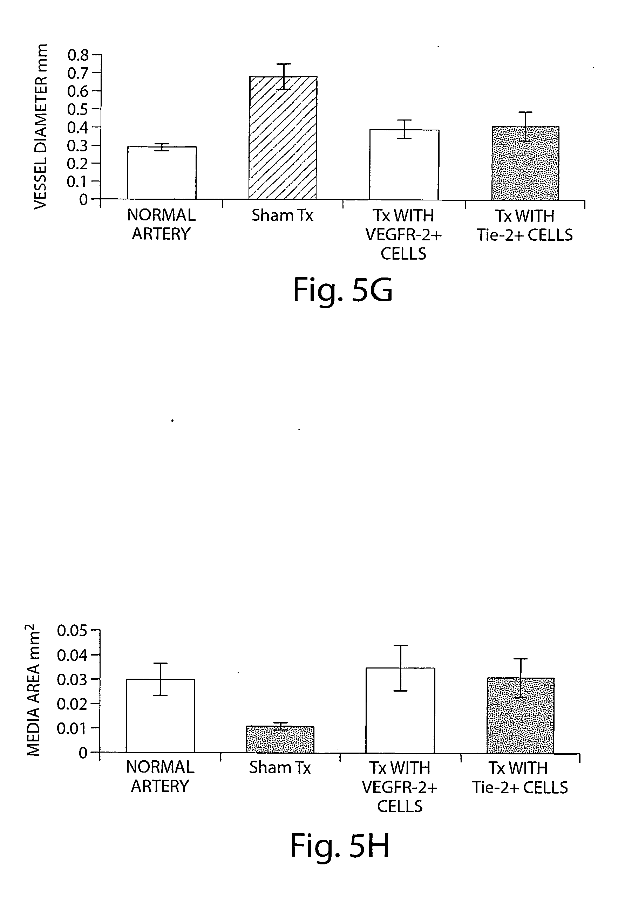

Figures 5 G-H are bar charts showing that the balloon-injury procedure in the mouse femoral artery resulted in dilation of the lesioned arterial segment in injured vessels incubated with media alone, but not in, femoral arteries of mice that had received transplantation of human cells. H, A reduction in cross-sectional media area from was observed in injured arteries, but not in VEGFR-2 and Tie-2 cells transplanted arteries.

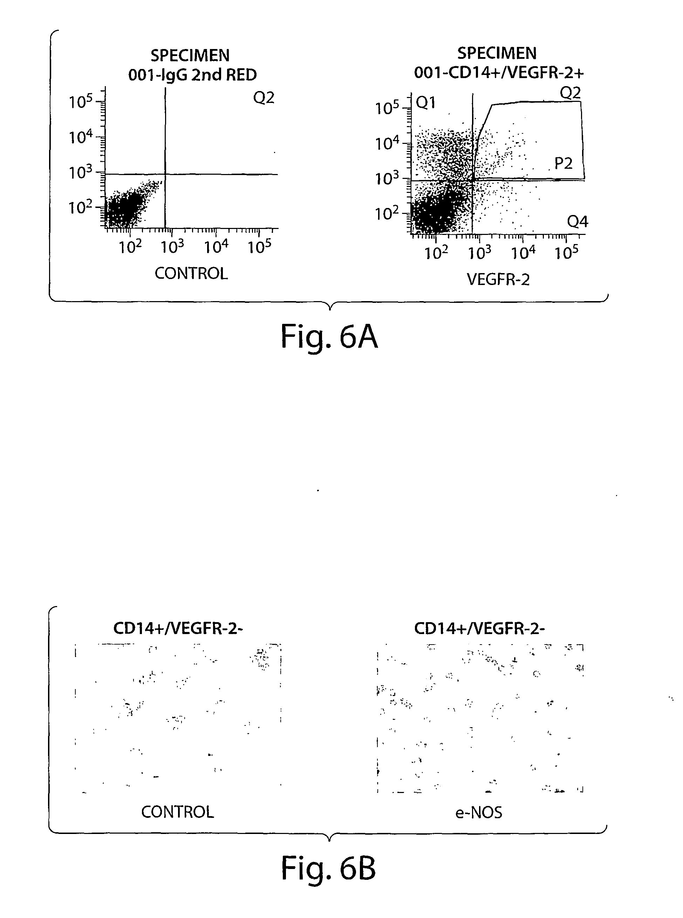

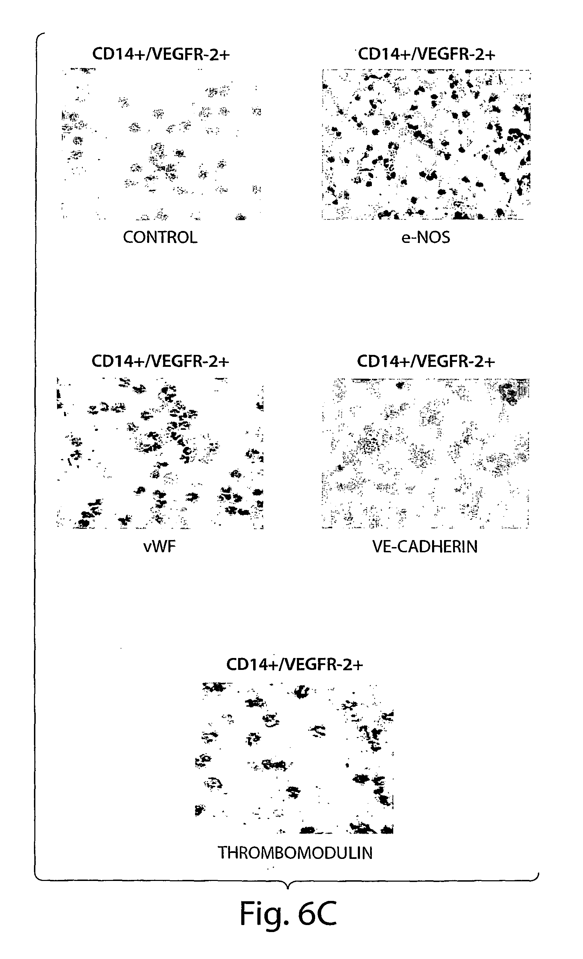

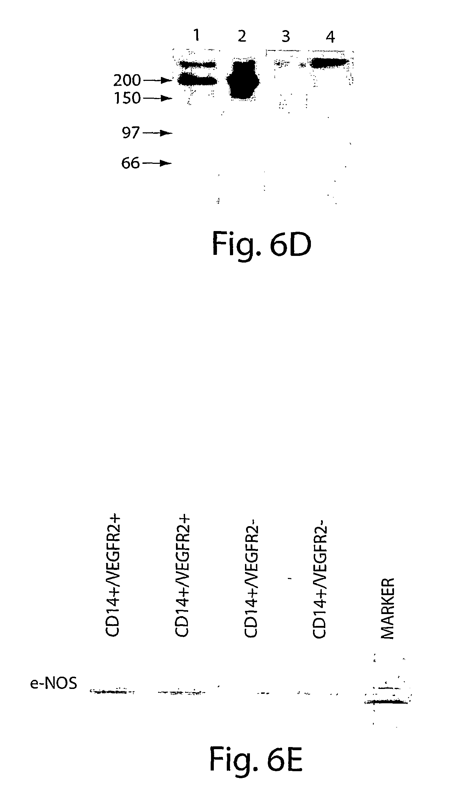

Figure 6 is a series of graphs and photographs showing the characterization of CD14+/VEGFR-2+ and CD14+/VEGFR-2- cells. **A,** Flow cytometry sorting of CD14+/VEGFR-2+ and CD14+/VEGFR-2- from peripheral blood mononuclear cells depleted of T lymphocytes. **B,** CD14+/VEGFR-2- cells did not express endothelial nitric oxide syntahse (e-NOS). **C,** CD14+/VEGFR-2+ cells expressed several endothelial-specific markers such as e-NOS (Black), von WilleBrand Factor (red) and thrombomodulin (red) but not VE-Cadherin. The cells were counterstained with hematoxylin. **D,** Western blotting of the lysates from CD14+/VEGFR-2+ cells with antibodies to VEGFR-2 gave the expected bands of app. 200kDa (Lanes 1&2), while CD14+/VEGFR-2- cells (Lane 3) and the control secondary antibody did not (Lane 4). **E,** A strong mRNA expression for e-NOS was detected in CD14+/VEGFR-2+ cells and weakly in the CD14+/VEGFR-2- cells.

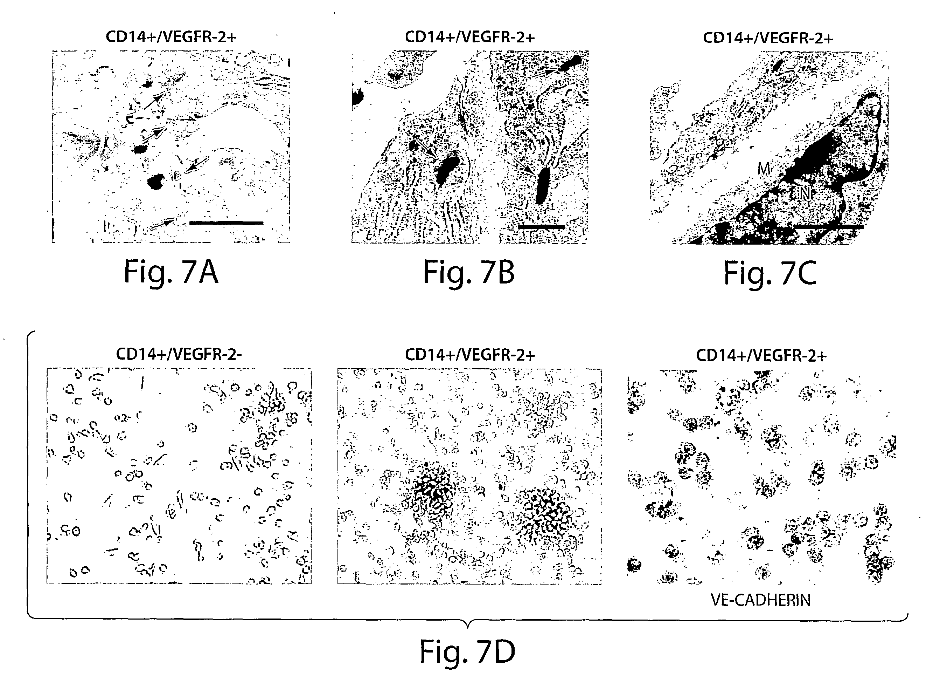

Figure 7 is series of photographs showing the *in vitro* morphological characterization of CD14+/VEGFR-2+ and CD14+/VEGFR-2- cells. **A,** TEM micrographs illustrate that CD14+/VEGFR-2+ cells demonstrated the presence of tight junctions typical for endothelial cells (arrows, bar;2µm), **B,** Weibel-Palade bodies (arrow, bar; 0.5µm) and **C,** produced basement membrane (M, bar; 1µm). **D,** CD14+/VEGFR-2- cells in culture did not form colonies while the CD14+/VEGFR-2+ cells formed colonies consisting of rounded cells initially which after one week grew out as single elongated cells. After one week in culture only the CD14+/VEGFR-2+ cells expressed VE-Cadherin..

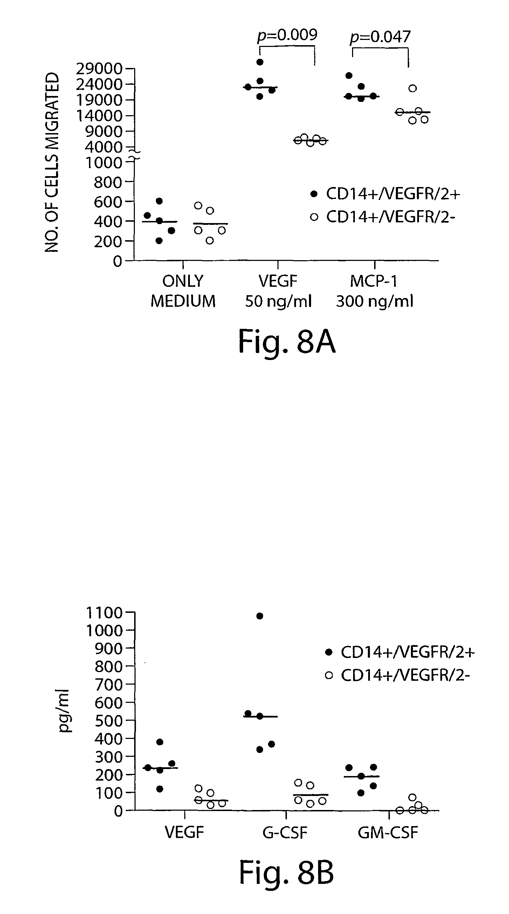

Figures 8 A and B are scatter graphs showing *in vitro* migratory and tubule-forming capacity of CD14+/VEGFR-2+ and CD14+/VEGFR-2- cells. **A,** In a migration assay in the absence and presence of 50 ng/ml concentration of vascular endothelial growth factor (VEGF) and 300 ng/ml monocyte chemotactic protein-1 (MCP-1) a significantly higher number of CD14+/VEGFR-2+ cells migrated towards VEGF and MCP-1 as compared to CD14+/VEGFR-2- cells. **B,** CD14+/VEGFR-2+ cell population produced higher levels of the angiogenic growth factors, VEGF, granulocyte colony-stimulating factor (G-CSF) and granulocyte-macrophage colonystimulating factor (GM-CSF) as compared to the CD14+/VEGFR-2- population.

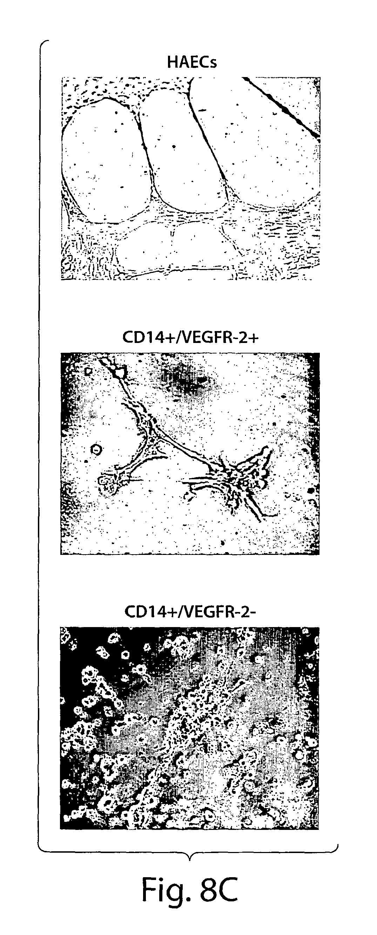

Figure 8 C is a series of photographs showing that CD14+/VEGFR-2+ but not CD14+/VEGFR-2-cells formed capillary like tubules in matrigel. Human aortic cells (HAECs) were used as control cells.

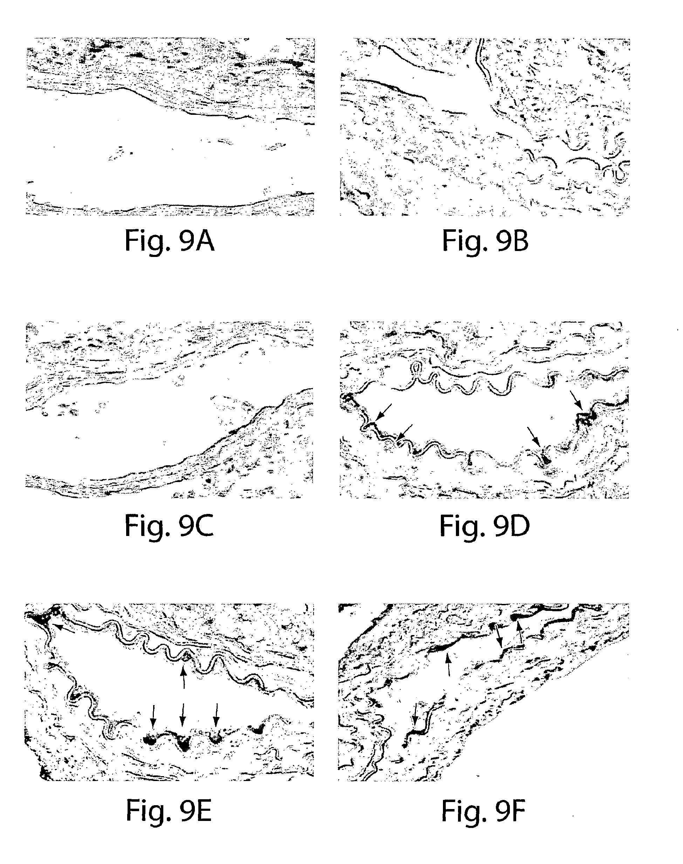

Figure 9 is a series of photographs showing *in vivo* engraftment capacity of CD14+/VEGFR-2+ and CD14+/VEGFR-2- cells. Immunohistochemical staining of sections from balloon-injured mouse femoral artery with an anti-green fluorescence protein (GFP) antibody showed **A,** no localization of GFP-positive areas in sham-transplanted and **B,** CD14+/VEGFR-2-transplanted cells in the luminal surface of the seeded artery. **C,** section showing staining with control secondary antibody. **D,** a significant number of GFP-positive (red) CD14+/VEGFR-2+ cells (arrows) at 2 weeks and **E&F,** at 4 weeks were found in the animals transplanted with these cells. Original magnification 60x.

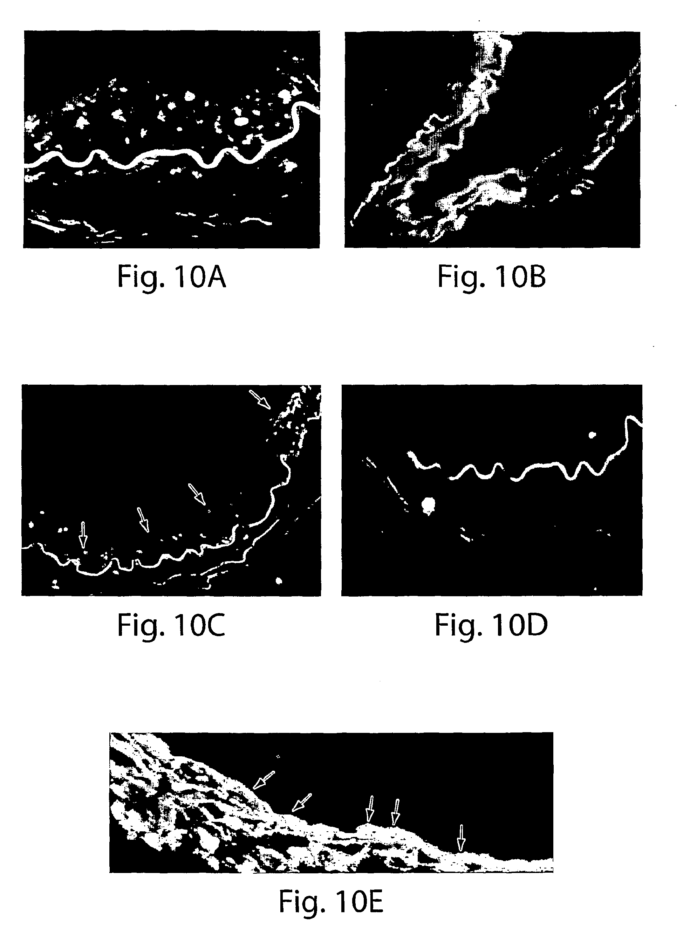

Figure 10 is a series of photographs showing immunofluorescence staining for detection of CD14+/VEGFR-2+ and CD14+/VEGFR-2- cells after transplantation. The human-nuclei antibody stained cells (red) in the **A,** normal human artery, **B,** but not control injured sham-transplanted mouse artery. **C,** Balloon-injured mouse femoral artery showed the localization of transplanted human CD14+/VEGFR-2+cells in the luminal surface of the seeded artery (red cells, arrows) at 4 weeks. **D,** However, no localization of transplanted CD14+/VEGFR-2- cells was observed at 4 weeks. **E,** The endothelial cell phenotype of transplanted human cells was confirmed by double staining with human-nuclei antibody and antibodies to VE-Cadherin (double positive cells are stained yellow, arrows) Original magnification 60x.

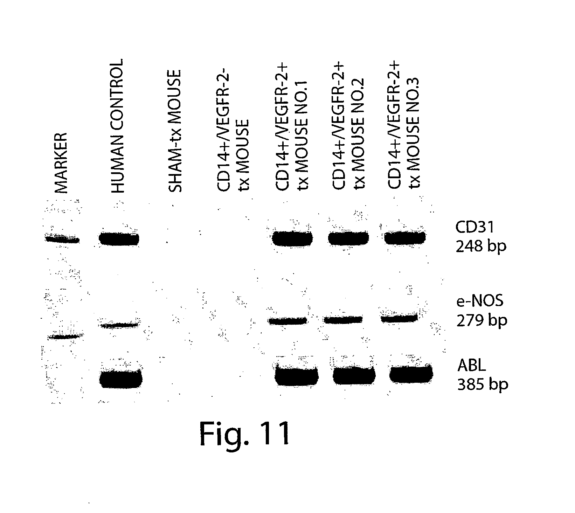

Figure 11 is a photograph of a Western Blot showing *in vivo* engraftment and functional capacity of CD14+/VEGFR-2+ and CD14+/VEGFR-2- cells. Transcription of human endothelial-specific genes in the mouse artery. Human CD31 and e-NOS were detected in balloon-injured mouse femoral arteries that received human CD14+/VEGFR-2+cells but not in the sham or CD14+/VEGFR-2- cell transplanted mice. ABL was used as the house-keeping gene.

### DETAILED DESCRIPTION

The present invention is based upon the unexpected discovery of a defined population of progenitor cells within human peripheral blood that can be cultivated *in vitro* and maintain a progenitor phenotype. Moreover, when these cells are cultivated in vitro on matrigel they are capable of forming tubule-like structures. These cells, when transplanted into animals with arterial injury exhibited functional differentiation into endothelial cells and/or smooth muscle cells.

Vascular diseases include a wide spectrum of both acute and chronic conditions associated with significant morbidity and mortality world-wide. Endothelial cell transplantation has tremendous therapeutic potential in the treatment of tissues damaged by ischemia. Vascular disease includes any condition that affects the circulatory system. This ranges from diseases of the arteries, veins and lymph vessels to blood disorders that affect circulation. Vascular disease include of example, Peripheral Artery Disease, Aneurysm, Renal (Kidney) Artery Disease, Raynaud's Phenomenon, Buerger's Disease, Peripheral Venous Disease, Varicose Veins, Blood Clots In the Veins.

Coronary disorders, can be categorized into at least two groups. Acute coronary disorders include myocardial infarction, and chronic coronary disorders include chronic coronary ischemia, arteriosclerosis, congestive heart failure, angina, atherosclerosis, and myocardial hypertrophy. Other coronary disorders include stroke, myocardial infarction, dilated cardiomyopathy, restenosis, coronary artery disease, heart failure, arrhythmia, angina, or hypertension.

Acute coronary disorders result in a sudden blockage of the blood supply to the heart which deprives the heart tissue of oxygen and nutrients, resulting in damage and death of the cardiac tissue. In contrast, chronic coronary disorders are characterized by a gradual decrease of oxygen and blood supply to the heart tissue overtime causing progressive damage and the eventual death of cardiac tissue. Successful *in vitro and in vivo* expansion and differentiation of endothelial progenitor cells are useful for endothelial cell transplantation, and a vehicle for gene therapy.

The present invention provides methods for producing multipotent endothelial progenitor cells from peripheral blood that are capable of differentiating into endothelial cells and/or smooth muscle cells *in vivo*.

### Endothelial Progenitor Cells

The invention discloses endothelial progenitor cells (referred to herein as EPC) An EPC cell is an undifferentiated cell that can be induced to proliferate using the methods of the present invention. The EPC is capable of self-maintenance, such that with each cell division, at least one daughter cell will also be a EPC cell. EPCs are capable of being expanded 100, 250, 500, 1000, 2000, 3000, 4000, 5000 or more fold.

Phenotyping of EPCs reveal that these cells express the committed hematopoietic marker CD45. Additionally, an EPC is immunoreactive for VEGFR-2 and/or Tie-2, and non immunoreactive for Cadherin 5 (CD144). Optionally, the EPC is immunoreactive for CD14. EPCs that are not immunoreactive for CD14 are capable of differentiating *in vivo* to endothelial cells or smooth muscle cells. Alternatively, cells that are immunoreactive for CD14 are capable of only differentiating to endothelial cells. The EPC is a multipotent progenitor cell. By mutipotent progenitor cell is meant that the cell is capable of differentiating into more that one cell type. For example, the cell is capable of differentiating into a endothelial cell or a smooth muscle cell.

Vascular endothelial growth factor (VEGF) acts through specific tyrosine kinase receptors that includes VEGFR-1 (flt-1) and VEGFR-2 (flk-1/KDR) and VEGFR-3/Flt-4 which convey signals that are essential for embryonic angiogenesis and hematopoiesis. While VEGF binds to all three receptors, most biological functions are mediated via VEGFR-2 and the role of VEGFR-1 is currently unknown. VEGFR3/Flt4 signaling is known to be important for the development of lymphatic endothelial cells and VEGFR3 signaling may confer lymphatic endothelial-like phenotypes to endothelial cells. VEGFRs relay signals for processes essential in stimulation of vessel growth, vasorelaxation, induction of vascular permeability, endothelial cell migration, proliferation and survival. Endothelial cells express all different VEGF-Rs. During embryogenesis, it has been reported that a single progenitor cell, the hemangioblast can give rise to both the hematopoietic and vascular systems.

Tie-2 is an endothelial-specific receptor tyrosine kinase and a receptor for angiopoietin 1. It is a type I membrane protein that is expressed predominantly in the endothelium of actively growing blood vessels and may represent the earliest mammalian endothelial cell lineage marker. Tie-2 is likely involved in the regulation of endothelial cell proliferation and differentiation and may direct the special orientation of endothelial cells during the formation of blood vessels.

The CD14 antigen is a high affinity receptor for the complex of lipopolysaccharids (LPS) and LPS-Binding protein (LBP). The CD14 antigen is part of the functional heteromeric LPS receptor complex comprised of CD14, TLR4 and MD-2. CD14 is strongly expressed on most human monocytes and macrophages in peripheral blood, other body fluids and various tissues, such as lymph nodes and spleen. CD14 is weakly expressed on subpopulations of human neutrophils and myeloid dendritic cells.

The CD45 antigen is a tyrosine phosphatase, also known as the leukocyte common antigen (LCA). CD45 is present on all human cells of hematopoietic origin, except erythroid cells, platelets and their precursor cells. The CD45 molecule is required for T cell and B cell activation and is expressed in at least 5 isoforms, depending on the activation status of the cell.

CD 144, also called Cadherin 5 or VE-Cadherin is a 140 kDa protein belonging to the cadherin family of cell adhesion molecules. CD144 antigen is specific for endothelial cells and is located at the intercellular cleft sites of the junction within endothelial tissue. The cadherin 5 molecule may play a role in the permeability properties of vascular endothelium.

The blood can be obtained from any animal such as , fish, reptiles, birds, amphibians, and mammals, e.g. preferably rodents, and such as mice and humans.

VEGFR-1+, VEGFR-2+ and Tie-2+ cells constituted approximately 3.0±0.2%, 0.8±0.5%, 2.0±0.3% of the total population of mononuclear cells in blood respectively. CD14+/VEGFR-2+ cells constituted approximately 2.0±0.5% of the total population of monocytes and 0.08±0.04% of mononuclear cells in blood.

EPCs can be maintained *in vitro* in long-term cultures. The EPCs are capable of being passed in culture 2, 3, 4, 5, 6, 7, 8, 9, 10, 11,12 or more times.

### Isolating Endothelial Progenitor Cells

The disclosure relates to a method of isolating populations of endothelial, or smooth muscle progenitor cells. The population of cells are isolated by means of positive selection, or by a mixture of both positive and negative selection in either order. The population of progenitor cells is purified. A purified population of EPCs contains a significantly higher proportion of EPCs than the crude population of cells from which the cells are isolated.

For example, the purification procedure should lead at least to a five fold increase, preferably at least a ten fold increase, more preferably at least a fifteen fold increase, most preferably at least a twenty fold increase, and optimally at least a twenty-five fold increase in EPCs with respect to the total population. The purified population of EPC should include at least 15%, preferably at least 20%, more preferably at least 25%, most preferably at least 35%, and optimally at least 50% of EPCs.

The methods described herein can lead to mixtures comprising up to 75%, preferably up to 80%, more preferably up to 85%, most preferably up to 90% and optimally up to 95% of stem cells. Such methods are capable of producing mixtures comprising 99%, 99.90% and even 100% of EPCs. Accordingly, the purified populations of the invention contain significantly higher levels of EPCs than those that exist in nature, as described above.

The purified population of EPCs are isolated by contacting a crude mixture of cells containing a population of stem cells that express an antigen characteristic of the EPCs with a molecule that binds specifically to the extracellular portion of the antigen. Such a technique is known as positive selection. The binding of the EPCs to the molecule permit the EPCs to be sufficiently distinguished from contaminating cells that do not express the antigen to permit isolating the stem cells from the contaminating cells. The antigen is preferably VEGFR, and more preferably VEGFR-2.

The molecule used to separate progenitor cells from the contaminating cells can be any molecule that binds specifically to the antigen that characterizes the EPCs. The molecule can be, for example, a monoclonal antibody, a fragment of a monoclonal antibody, or, in the case of an antigen that is a receptor, the ligand of that receptor. For example, in the case of a VEGF receptor, such as FLK-1, the ligand is VEGF.

The unique isolated cells of the present disclosure are separated from other cells by virtue of their CD45+ state and possession of vascular endothelial growth factor receptors (VEGFR), e.g. VEGFR-2. The cells can be isolated by conventional techniques for separating cells, such as those described in Civin, U.S. Pat. Nos. 4,714,680, 4,965,204, 5,035,994, and 5,130,144, Tsukamoto et al U.S. Pat. No. 5,750,397, and Loken et al, U.S. Pat. No. 5,137,809. Thus, for example, a CD45 specific monoclonal antibody or an VEGFR-specific antibody can be immobilized on a solid support such as nitrocellulose, agarose beads, polystyrene beads, hollow fiber membranes, magnetic beads, and plastic petri dishes. The entire cell population is then be passed through the solid support or added to the beads.

Cells that are bound to the binding molecule are removed from the cell suspension by physically separating the solid support from the remaining cell suspension. For example, the unbound cells may be eluted or washed away with physiologic buffer after allowing sufficient time for the solid support to bind the stem cells.

The bound cells are separated from the solid phase by any appropriate method, depending mainly upon the nature of the solid phase and the binding molecule. For example, bound cells can be eluted from a plastic petri dish by vigorous agitation. Alternatively, bound cells can be eluted by enzymatically "nicking" or digesting an enzyme-sensitive "spacer" sequence between the solid phase and an antibody. Suitable spacer sequences bound to agarose beads are commercially available from, for example, Pharmacia.

The eluted, enriched fraction of cells may then be washed with a buffer by centrifugation and preserved in a viable state at low temperatures for later use according to conventional technology. The cells may also be used immediately, for example by being infused intravenously into a recipient.

Those which remain attached to the solid support are those cells which contain a marker which is recognized by the antibody used. Thus, if the anti-CD45 antibody is used, then the resulting population will be greatly enriched in CD45⁺ cells. If the antibody used is VFGFR, then the resulting population will be greatly enriched in VEGFR⁺ cells. That population may then be enriched in the other marker by repeating the steps using a solid phase having attached thereto an antibody to the other marker.

Another way to sort CD45⁺ VEGFR⁺ cells is by means of flow cytometry, most preferably by means of a fluorescence-activated cell sorter (FACS), such as those manufactured by Becton-Dickinson under the names FACScan or FACSCalibur. By means of this technique, the cells having a CD45 marker thereon are tagged with a particular fluorescent dye by means of an anti-CD45antibody which has been conjugated to such a dye. Similarly, the VEGFR marker of the cells are tagged with a different fluorescent dye by means of an anti-VEGFR antibody which is conjugated to the other dye. When the stained cells are placed on the instrument, a stream of cells is directed through an argon laser beam that excites the fluorochrome to emit light. This emitted light is detected by a photomultiplier tube (PMT) specific for the emission wavelength of the fluorochome by virtue of a set of optical filters. The signal detected by the PMT is amplified in its own channel and displayed by a computer in a variety of different forms-e.g., a histogram, dot display, or contour display. Thus, fluorescent cells which emit at one wavelength, express a molecule that is reactive with the specific fluorochrome-labeled reagent, whereas non-fluorescent cells or fluorescent cells which emit at a different wavelength do not express this molecule but may express the molecule which is reactive with the fluorochrome-labeled reagent which fluoresces at the other wavelength. The flow cytometer is also semi-quantitative in that it displays the amount of fluorescence (fluorescence intensity) expressed by the cell. This correlates, in a relative sense, to the number of the molecules expressed by the cell.

Flow cytometers are also equipped to measure non-fluorescent parameters, such as cell volume or light scattered by the cell as it passes through the laser beam. Cell volume is usually a direct measurement. The light scatter PMTs detect light scattered by the cell either in a forward angle (forward scatter; FSC) or at a right angle (side scatter; SSC). FSC is usually an index of size, whereas SSC is an index of cellular complexity, although both parameters can be influenced by other factors.

Preferably, the flow cytometer is equipped with more than one PMT emission detector. The additional PMTs may detect other emission wavelengths, allowing simultaneous detection of more than one fluorochrome, each in individual separate channels. Computers allow the analysis of each channel or the correlation of each parameter with another. Fluorochromes which are typically used with FACS machines include fluorescein isothiocyanate (FITC), which has an emission peak at 525 nm (green), R-phycoerythrin (PE), which has an emission peak at 575 nm (orange-red), propidium iodide (PI), which has an emission peak at 620 nm (red), 7-aminoactinomycin D (7-AAD), which has an emission peak at 660 nm (red), R-phycoerythrin Cy5 (RPE-Cy5), which has an emission peak at 670 nm (red), and allophycocyanin (APC), which has an emission peak at 655-750 nm (deep red).

These and other types of FACS machines may have the additional capability to physically separate the various fractions by deflecting the cells of different properties into different containers.

Any other method for isolating the CD45⁺ VEGFR⁺ population of a starting material, such as bone marrow, peripheral blood or cord blood, may also be used in accordance with the present invention. The various subpopulations (e.g., CD14⁺, Tie2⁺, CD144⁻) of the present invention may be isolated in similar manners.

Either before or after the crude cell populations are purified as described above, the population of progenitor cells may be further concentrated by methods known in the art. For example, the progenitor cells can be enriched by positive selection for one or more antigens characteristic of EPCs. Such antigens include, for example, CD 14 or Tie-2.

In a particularly preferred variation of the method described above, blood is withdrawn directly from the circulating peripheral blood of a donor. The blood is percolated continuously through a column containing the solid phase-linked binding molecule, such as an antibody VEGFR-2, to capture EPCs. The progenitor cell-depleted blood is returned immediately to the donor's circulatory system by methods known in the art, such as hemapheresis. The blood is processed in this way until a sufficient number of progenitor cells binds to the column. The stem cells are then isolated from the column by methods known in the art. This method allows rare peripheral blood progenitor cells to be harvested from a very large volume of blood, sparing the donor the expense and pain of harvesting bone marrow and the associated risks of anesthesia, analgesia, blood transfusion, and infection.

### Culture conditions

EPCs are cultivated and proliferated using the methods described herein. Cells are obtained peripheral blood by isolating peripheral blood mononuclar cells (PBMC) by density gradient centrifugation.

Cell suspensions are seeded in any receptacle capable of sustaining cells, particularly culture flasks, culture plates or roller bottles, and more particularly in small culture flasks such as 25 cm² culture flasks. Cells cultured in suspension are resuspended at approximately 5 x 10⁴ to 2 x 10⁵ cells/ml (*for example*, 1 x 10⁵ cells/ml). Cells plated on a fixed substrate are plated at approximately 2-3 x 10³ 10 cells/cm². Optionally, the culture plates are coated with a matrix protein such as collagen. The cells can be placed into any known culture medium capable of supporting cell growth, including HEM, DMEM, RPMI, F-12, and the like, containing supplements which are required for cellular metabolism such as glutamine and other amino acids, vitamins, minerals and proteins such as transferrin and the like. The culture medium may also contain antibiotics to prevent contamination with yeast, bacteria and fungi such as penicillin, streptomycin, gentamicin and the like. The culture medium may contain serum derived from bovine, equine, chicken and the like.

Conditions for culturing should be close to physiological conditions. The pH of the culture medium should be close to physiological pH. (*for example*, between pH 6-8, between about pH 7 to 7.8, or at pH 7.4). Physiological temperatures range between about 30°C to 40°C. EPCs are cultured at temperatures between about 32°C to about 38°C (*for example*, between about 35°C to about 37°C).

Optionally, the culture medium is supplemented with at least one proliferation-inducing ("mitogenic") growth factor. A "growth factor" is protein, peptide or other molecule having a growth, proliferation-inducing, differentiation-inducing, or trophic effect on EPCs. "Proliferation-inducing growth factors" are trophic factor that allows EPCs to proliferate, including any molecule that binds to a receptor on the surface of the cell to exert a trophic, or growth-inducing effect on the cell. Proliferation-inducing growth factors include EGF, amphiregulin, acidic fibroblast growth factor (aFGF or FGF-1), basic fibroblast growth factor (bFGF or FGF-2), transforming growth factor alpha (TGFα), VEGF and combinations thereof. Growth factors are usually added to the culture medium at concentrations ranging between about 1 fg/ml to 1 mg/ml. Concentrations between about 1 to 100 ng/ml are usually sufficient. Simple titration assays can easily be performed to determine the optimal concentration of a particular growth factor.

The biological effects of growth and trophic factors are generally mediated through binding to cell surface receptors. The receptors for a number of these factors have been identified and antibodies and molecular probes for specific receptors are available. EPCs can be analyzed for the presence of growth factor receptors at all stages of differentiation. In many cases, the identification of a particular receptor provides guidance for the strategy to use in further differentiating the cells along specific developmental pathways with the addition of exogenous growth or trophic factors.

Generally, after about 3-10 days *in vitro,* the culture medium of EPCs is replenished by aspirating the medium, and adding fresh medium to the culture flask. Optionally, the aspirated medium is collected, filtered and used as a condition medium to subsequently passage EPCs. For example the 10%, 20%, 30%, 40% or more condition medium is used.

The EPC cell culture can be easily passaged to reinitiate proliferation. For example, after 3-7 days *in vitro,* the culture flasks are shaken well and EPCs are then transferred to a 50 ml centrifuge tube and centrifuged at low speed. the medium is aspirated, the EPCs are resuspended in a small amount of culture medium The cells are then counted and replated at the desired density to reinitiate proliferation. This procedure can be repeated weekly to result in a logarithmic increase in the number of viable cells at each passage. The procedure is continued until the desired number of EPCs is obtained.

EPCs and EPC progeny can be cryopreserved by any method known in the art until they are needed. (*See, e.g.,* United States patent 5,071,741, PCT International patent applications WO93/14191, WO95/07611, WO96/27287, WO96/29862, and WO98/14058, Karlsson et al., 65 Biophysical J. 2524-2536 (1993)). The EPCs can be suspended in an isotonic solution, preferably a cell culture medium, containing a particular cryopreservant. Such cryopreservants include dimethyl sulfoxide (DMSO), glycerol and the like. These cryopreservants are used at a concentration of 5-15% (*for example,* 8-10%). Cells are frozen gradually to a temperature of -10°C to -150°C (*for example*, -20°C to -100°C, or -70°C to -80°C).

### Differentiation of Endothelial Progenitor Cells

Depending on the culture conditions, EPCs can be differentiated into endothelial cells or smooth muscle cells.

EPCs can be differentiated into endothelial cells or smooth muscle cells EPCs on a fixed substrate in a culture medium with a differentiation-inducing growth factor. Differentiation of the EPCs can also be induced by any method known in the art which activates the cascade of biological events which lead to growth, which include the liberation of inositol triphosphate and intracellular Ca²⁺, liberation of diacyl glycerol and the activation of protein kinase C and other cellular kinases, and the like. Treatment with phorbol esters, differentiation-inducing growth factors and other chemical signals can induce differentiation. Instead of proliferation-inducing growth factors for the proliferation of EPCs (*see above*), differentiation-inducing growth factors can be added to the culture medium to influence differentiation of the EPCs. Other differentiation inducing growth factors include platelet derived growth factor (PDGF), thyrotropin releasing hormone (TRH), transforming growth factor betas (TGF,s), insulin-like growth factor (IGF-1) and the like.

Differentiated endothelial cells or smooth muscle cells are detected using immunocytochemical techniques know in the art. Immunocytochemistry (*e.g.* dual-label immunofluorescence and immunoperoxidase methods) uses antibodies that detect cell proteins to distinguish the cellular characteristics or phenotypic properties of endothelial cells or smooth muscle cells Cellular markers for endothelial cells include for example, VE-cadherin, CD144, CD141, CD 106, or CD142 whereas cellular markers for smooth muscle cells includes Flk. Immunocytochemistry can also be used to identify endothelial cells, by detecting the expression of endothelial cell genes such as CD31 and e-NOS.

*In situ* hybridization histochemistry can also be performed, using cDNA or RNA probes specific for the endothelial gene mRNAs. These techniques can be combined with immunocytochemical methods to enhance the identification of specific phenotypes. If necessary, the antibodies and molecular probes discussed above can be applied to Western and Northern blot procedures respectively to aid in cell identification.

### Transplantation of Endothelial Progenitor Cells

Transplantation of new cells into the damaged blood vessels has the potential to repair damaged vascular tissue, e.g., veins, arteries, capillaries, thereby restoring vascular function. However, the absence of suitable cells for transplantation purposes has prevented the full potential of this procedure from being met. "Suitable" cells are cells that meet one or more of the following criteria: (1) can be obtained in large numbers; (2) can be proliferated *in vitro* to allow insertion of genetic material, if necessary; (3) capable of surviving indefinitely and facilitate vascular repair on transplantation r; and (4) are non-immunogenic, preferably obtained from a patient's own tissue or from a compatible donor. The use of EPCs in the treatment of vascular disorders can be demonstrated by the use of animal models.

EPCs are administered to any animal with abnormal vasculature or coronary failure symptoms. EPCs can be prepared from donor tissue that is xenogeneic to the host. For xenografts to be successful, some method of reducing or eliminating the immune response to the implanted tissue is usually employed. Thus EPCs recipients can be immunosuppressed, either through the use of immunosuppressive drugs such as cyclosporin, or through local immunosuppression strategies employing locally applied immunosuppressants. Local immunosuppression is disclosed by Gruber, 54 Transplantation 1-11(1992). United States patent 5,026,365 discloses encapsulation methods suitable for local immunosuppression.

As an alternative to employing immunosuppression techniques, methods of gene replacement or knockout using homologous recombination in embryonic stem cells, taught by Smithies *et al*., 317 Nature 230-234 (1985), and extended to gene replacement or knockout in cell lines (Zheng *et al*., 88 Proc. Natl. Acad. Sci. 8067-8071 (1991)), can be applied to EPCs for the ablation of major histocompatibility complex (MHC) genes. EPCs lacking MHC expression allows for the grafting of enriched endothelial cell populations across allogeneic, and perhaps even xenogeneic, histocompatibility barriers without the need to immunosuppress the recipient. General reviews and citations for the use of recombinant methods to reduce antigenicity of donor cells are also disclosed by Gruber, 54 Transplantation 1-11(1992). Exemplary approaches to the reduction of immunogenicity of transplants by surface modification are disclosed by PCT International patent application WO 92/04033 and PCT/US99/ 24630. Alternatively the immunogenicity of the graft may be reduced by preparing EPCs from a transgenic animal that has altered or deleted MHC antigens.

EPCs can be encapsulated and used to deliver factors to the host, according to known encapsulation technologies, including microencapsulation (see, *e.g.*, United States patents 4,352,883; 4,353,888; and 5,084,350) and macroencapsulation (*see, e.g.,* United States patents 5,284,761, 5,158,881, 4,976,859 and 4,968,733 and PCT International patent applications WO 92/19195 and WO 95/05452). Macroencapsulation is described in United States patents 5,284,761; 5,158,881; 4,976,859; 4,968,733; 5,800,828 and PCT International patent application WO 95/05452. Cell number in the devices can be varied; preferably each device contains between 10³ - 10⁹ cells (for example, 10⁵ to 10⁷ cells). Multiple macroencapsulation devices can be implanted in the host.

EPCs prepared from tissue that is allogeneic to that of the recipient is tested for use by the well-known methods of tissue typing, to closely match the histocompatibility type of the recipient.

EPCs can sometimes be prepared from the recipient's own blood or bone marrow. In such instances the EPCs can be generated from dissociated tissue and proliferated *in vitro* using the methods described above. Upon suitable expansion of cell numbers, the EPCs may be harvested, genetically modified if necessary, and readied for direct injection into the recipient's vasculature

EPCs are administered to the vasculature can form a vascular graft, so that the cells form normal connections with neighboring vascular cells , maintaining contact with transplanted or existing endothelial cells. Thus the transplanted EPCs re-establish the vascular tissue which have been damaged due to disease and aging.

Functional integration of the graft into the host's vascular tissue can be assessed by examining the effectiveness of grafts on restoring various functions.

The ability to expand EPCs *in vitro* for use in transplantation is also useful for *ex vivo* gene therapy. Thus, EPCs provide an additional way to retrieve and expand endothelial cells for use as vehicles in *ex vivo* gene therapy trials.

### Genetic Modification of Endothelial Progenitor Cells

Although the EPCs are non-transformed primary cells, they possess features of a continuous cell line. In the undifferentiated state, the EPCs continuously divide and are thus targets for genetic modification. In some embodiments, the genetically modified cells are induced to differentiate into endothelial cells or smooth muscle cells by any of the methods described above.

The term "genetic modification" refers to the stable or transient alteration of the genotype of a EPCs by intentional introduction of exogenous DNA. DNA may be synthetic, or naturally derived, and may contain genes, portions of genes, or other useful DNA sequences. The term "genetic modification" as used herein is not meant to include naturally occurring alterations such as that which occurs through natural viral activity, natural genetic recombination, or the like.

Any useful genetic modification of the cells is within the scope of the present invention. For example, EPCs may be modified to produce or increase production of a biologically active substance such as a growth factor or the like. In one embodiment the biologically active substance is a transcription factor such as a transcription factor that modulates genetic differentiation. In an alternative embodiment the biologically active substance is a non-mitogenic proliferation factor, *e.g.* v-myc, SV-40 large T or telomerase.

The genetic modification is performed either by infection with viral vectors (retrovirus, modified herpes viral, herpes-viral, adenovirus, adeno-associated virus, and the like) or transfection using methods known in the art (lipofection, calcium phosphate transfection, DEAE-dextran, electroporation, and the like) (*see,* Maniatis et al., in Molecular Cloning: A Laboratory Manual (Cold Spring Harbor Laboratory, N.Y., 1982)). For example, the chimeric gene constructs can contain viral, for example retroviral long terminal repeat (LTR), simian virus 40 (SV40), cytomegalovirus (CMV); or mammalian cell-specific promoters such as tyrosine hydroxylase (TH, a marker for dopamine cells), DBH, phenylethanolamine N-methyltransferase (PNMT), ChAT, GFAP, NSE, the NF proteins (NE-L, NF-M, NF-H, and the like) that direct the expression of the structural genes encoding the desired protein. In addition, the vectors can include a drug selection marker, such as the *E. coli* aminoglycoside phosphotransferase gene, which when co-infected with the test gene confers resistance to geneticin (G418), a protein synthesis inhibitor.

EPCs can be genetically modified using transfection with expression vectors. In one protocol, vector DNA containing the genes are diluted in 0.1X TE (1 mM Tris pH 8.0, 0.1 mM EDTA) to a concentration of 40 µg/ml. 22 µl of the DNA is added to 250 µl of 2X HBS (280 mM NaCl, 10 mM KCl, 1.5 mM Na₂HPO₄, 12 mM dextrose, 50 mM HEPES) in a disposable, sterile 5 ml plastic tube. 31 µl of 2 M CaCl₂ is added slowly and the mixture is incubated for 30 minutes (min) at room temperature. During this 30 min incubation, the cells are centrifuged at 800 g for 5 min at 4°C. The cells are resuspended in 20 volumes of ice-cold PBS and divided into aliquots of 1x10⁷ cells, which are again centrifuged. Each aliquot of cells is resuspended in 1 ml of the DNA-CaCl₂ suspension, and incubated for 20 min at room temperature. The cells are then diluted in growth medium and incubated for 6-24 hr at 37°C in 5%-7% CO₂. The cells are again centrifuged, washed in PBS and returned to 10 ml of growth medium for 48 hr.

EPCs are also genetically modified using calcium phosphate transfection techniques. For standard calcium phosphate transfection, the cells are mechanically dissociated into a single cell suspension and plated on tissue culture-treated dishes at 50% confluence (50,000-75,000 cells/cm²) and allowed to attach overnight. In one protocol, the modified calcium phosphate transfection procedure is performed as follows: DNA (15-25 µg) in sterile TE buffer (10 mM Tris, 0.25 mM EDTA, pH 7.5) diluted to 440 µL with TE, and 60 µL of 2 M CaCl² (pH to 5.8 with 1M HEPES buffer) is added to the DNA/TE buffer. A total of 500 µL of 2x HeBS (HEPES-Buffered saline; 275 mM NaCl, 10 mM KCl, 1.4 mM Na₂ HPO₄, 12 mM dextrose, 40 mM HEPES buffer powder, pH 6.92) is added dropwise to this mix. The mixture is allowed to stand at room temperature for 20 min. The cells are washed briefly with 1x HeBS and 1 ml of the calcium phosphate precipitated DNA solution is added to each plate, and the cells are incubated at 37°C for 20 min. Following this incubation, 10 ml of medium is added to the cells, and the plates are placed in an incubator (37°C, 9.5% CO₂) for an additional 3-6 hours. The DNA and the medium are removed by aspiration at the end of the incubation period, and the cells are washed 3 times and then returned to the incubator.

When the genetic modification is for the production of a biologically active substance, the substance can be one that is useful for the treatment of a given vascular disorder. EPCs are be genetically modified to express a biologically active agent, such as growth factors, growth factor receptors. For example, it may be desired to genetically modify cells so they secrete a proliferation-inducing growth factor or a differentiation-inducing growth factor.

The genetically modified EPCs can be implanted for cell therapy or gene therapy into the CNS of a recipient in need of the biologically active molecule produced by the genetically modified cells. Transplantation techniques are detailed below.

Alternatively, the genetically modified EPCs can be subjected to various differentiation protocols *in vitro* prior to implantation. Once the cells have differentiated, they are again assayed for expression of the desired protein. Cells having the desired phenotype can be isolated and implanted into recipients in need of the protein or biologically active molecule that is expressed by the genetically modified cell.

The present invention is further illustrated, but not limited, by the following examples.

### EXAMPLE 1: GENERAL METHODS

### Isolation of VEGFR-1+, VEGFR-2+ and Tie-2+ cells from peripheral blood

Peripheral blood mononuclear cells (PBMC) expressing either VEGFR-1, VEGFR-2 or Tie-2 were obtained first by isolating mononuclear cells using Ficoll density-gradient centrifugation of human blood buffy coats. PBMC were distributed into several tubes each containing 40 x 10⁶ cells. 15µl of anti-VEGFR-1(4µg/ml), -VEGFR-2 (20µg/ml) or -Tie-2 (5µg/ml) antibodies (for details of antibodies see Table 1) were added to each tube and incubated for 30 min at room temperature. After washing once with phosphate buffered saline (PBS) the cells were labeled with 10µl of a 1:10 diluted secondary goat anti-mouse monoclonal antibody. Cells were sorted by fluorescence cytometry using a stringent gate to obtain highly purified populations of these cells.

### Isolation of CD14+/VEGFR-2+ and CD14+/VEGFR-2- cells from peripheral blood

Peripheral blood mononuclear cells (PBMC) were isolated by density-gradient centrifugation with Ficoll from blood of healthy human volunteers according to standard procedure. CD 14+/VEGFR-2+ cells were isolated by fluorescence-activated cell sorting. Staining was carried out as follows: PBMC were initially incubated with anti-CD3 antibody coated magnetic particles (DYNAL, Norway) to remove T lymphocytes. The procedure was carried out as described by the manufacturers. The remaining cells were distributed into several tubes each containing 20 x 10⁶ cells. 15 µl of anti-VEGFR-2 (20 µg/ml, Reliatech, Germany) antibodies were added to each tube and incubated for 30 min at room temperature. After washing once with phosphate buffered saline (PBS) the cells were labeled with 20 µl of a 1:10 diluted secondary FITC-conjugated goat anti-mouse monoclonal antibody (Serotec, Sweden) and 20 µl PE-conjugated anti-CD14 antibodies (4µg/ml, BD Biosciences, USA). During cell sorting, using a stringent gate, highly purified CD14+/VEGFR-2+ as well as the CD14+/VEGFR-2- fractions were collected for further analysis.

### Western blotting

Lysates of CD14+/VEGFR-2+ and CD14+/VEGFR-2- cells (1x10⁶ cells for each population) were separated by SDS-PAGE, transferred to nitrocellulose membranes and immunoblotted with anti-VEGFR-2 antibodies (1:200). Horseradish peroxidase conjugated F(ab')₂ fragments of goat anti-mouse secondary antibodies (1:2000) (Jackson Immunoresearch, USA) were used together with the ECL kit (Amersham) to identify the bound antibody.

### Flow Cytometry, Phenotyping and Cell Cultivation

Phenotyping of precursor endothelial cells was performed as described earlier¹⁵ using an array of antibodies to specific markers expressed on haematopoietic and endothelial cells (Table 1). Anti-VEGFR-2 was obtained from ReliaTech (Germany); -VEGFR-1 R&D (England); -AcLDL Molecular Probes (USA); -von Willebrand factor-HLA,-CD142 and - HLA class I DAKO (Denmark); -α-actin and -fibroblast antibody Serotec (Sweden); -CD144, -CD141, human nuclei antibody from Chemicon (Sweden); and Ulex europaeus Sigma (Sweden). All other antibodies were purchased from Becton Dickinson (USA). For certain markers intracellular staining was performed after cell permeabilization using 5% saponin in phosphate buffered saline (PBS). Corresponding control isotypes were used for evaluation of non-specific binding of monoclonal antibodies (Mabs). The cells were analysed on a Becton Dickinson flow cytometer (FACSorter). Sorted VEGFR-1+, VEGFR-2+ and Tie-2+ cells were cultivated on fibronectin -coated tissue culture plates, in endothelial selective medium MCDB 131 (GIBCO, Gaithersburg, MD, USA) containing 5 mM L-glutamine, and 100 µg/ml penicillin/streptomycin (PEST). The medium was further supplemented with endothelial cell growth medium (EGM-2) singlequots (Clonetics, BioWhittaker, USA). Human umbilical vein endothelial cells (HUVEC) were purchased from Clonetics and cultivated in recommended EGM-2-MV medium supplemented with EGM-2-MV singlequots. HUVEC were used as controls in the phenotypic analysis and the capillary formation assay.

FACS sorted cells were placed on glass slides using cytospin. Immunocytochemistry was performed by enzyme staining. The cells were stained with the following antibodies: anti-CD144 (VE-Cadherin), -CD141 (Thrombomodulin) both from Becton Dickinson, USA, -VEGFR-1 (R&D Systems, England) and antibodies to endothelial nitric oxide synthase (e-NOS) (all antibodies were used at a dilution of 1:50) for 1 hour at room temperature, washed three times with PBS and stained with secondary biotinylated goat anti-mouse antibodies (1:500). The avidin-peroxidase procedure was carried out using Vectastain Elite ABC kit (ImmunKemi, Stockholm, Sweden) as described by the manufacturers. The ACE (gives red staining) or DABNickel (gives brown/black staining) substrate kits were used as color developers. Since higher numbers of cells were obtained from the CD14+/VEGFR-2-population, these cells were further characterized by flow cytometry as described earlier¹⁶ using an array of antibodies to specific markers expressed on monocytes and endothelial cells (Table 3). FITC conjugated-acetylated low density lipoprotein (AcLDL) (Molecular Probes,USA), antibodies to-von Willebrand factor (DAKOPATTS, Denmark), α-actin, fibroblast (Serotec, Sweden), and the Ulex europaeus (Sigma, Sweden) were used. All other antibodies were purchased from Becton Dickinson (USA). Corresponding control isotypes were used for evaluation of non-specific binding of monoclonal antibodies (Mabs). The cells were analyzed on a Becton Dickinson flow cytometer (FACSorter).

For some experiments, sorted CD14+/VEGFR-2+ and CD14+/VEGFR-2- cells were cultivated on fibronectin (20 µg/ml )-coated tissue culture plates, in endothelial selective medium EndoCult™ (Stem Cell Technologies, Canada). These cultures were observed for growth and morphology. The cells after one week in culture were cytospinned on glass and stained for VE-Cadherin expression.

Human aortic endothelial cells (HAECs) were purchased from Clonetics and cultivated in recommended medium. HAECs were used as controls in the phenotypic analysis and the tubule formation assay.

### Transmission electron microscopy

Cells were grown on membrane filters in 24 well plates. Electron microscopic analysis was performed at the core facility unit for electron microscopy at Karolinska University hospital-Huddinge, Sweden. CD14+/VEGFR-2+ and CD14+/VEGFR-2- cells were grown separately on membrane filters in 24 well plates. The membranes were fixed in 2% glutaraldehyde, briefly rinsed in distilled water, placed in 70% ethanol for 10 min, and in 99.5% ethanol for 15 min, all at 4°C and dried. After fixation, the membrane was cut free, fixed for 1 hr at 4°C in a buffer containing 0.15 M sodium cacodylate, 1% osmium tetraoxide, and 3mM CaCl₂, pH 7.4. Subsequently, the wells were rinsed briefly, in 0.15 M sodium cacodylate buffer, dehydrated in ethanol as described above, and imbedded in Spurr resin (Agar Scientific LTD, Essex, England). The sections were contrasted with uranyl acetate followed by lead citrate, and examined at 80 kV in a Leo 906 (Oberkochen, Germany) transmission electron microscope.

### Migration assay

Freshly isolated VEGFR1+, VEGFR-2+ and Tie-2+ cells (5 x 10⁴) were added to four fibronectin-coated Transwell inserts having a 3.0µm pore size for each cell type. Precursor endothelial cell migration were assayed in the absence and presence of varying concentrations in the lower chamber of vascular endothelial growth factor (VEGF) 0ng/ml, 0.5ng/ml, 2ng/ml and 10ng/ml in medium containing 5% fetal calf serum. Cells were allowed to migrate for 22 hrs. Migrated cells were collected and counted in a haemocytometer. Similar experiments were performed using angiopoietin-2 (the ligand for Tie-2) at concentrations of 0ng/ml, 100ng/ml, 200ng/ml and 300ng/ml. Each experiment was performed in triplicate wells and repeated four times.

Freshly isolated CD14+/VEGFR-2+ and CD14+/VEGFR-2- cells (5 x 10⁴) were added to four fibronectin-coated Transwell inserts each, and all having a 3.0µm membrane pore size. The cell migration were assayed in the absence and presence of 50 ng/ml in the lower chamber of vascular endothelial growth factor (VEGF) or 300 ng/ml monocyte chemotactic factor-1 (MCP-1) in medium containing 5% fetal calf serum. Cells were allowed to migrate for 22 hrs. Migrated cells were collected and counted in a haemocytometer. Each experiment was performed in triplicate wells and repeated four times.

### Angiogenic growth factor secretion by CD14+/VEGFR-2+ and CD14+/VEGFR-2- cell populations

To assess growth factor secretion, freshly isolated cells were cultivated in growth factor- and serum-free medium for 72 hrs. Supernatants from all cultures were collected and stored at -70°C until further analysis. Conditioned medium were assayed for the angiogenic growth factors, VEGF, granulocyte colony-stimulating factor (G-CSF), granulocyte-macrophage colonystimulating factor (GM-CSF), fibroblast growth factor (FGF) and stromal derived-factor-1 (SDF-1). The growth factors were assayed by ELISA (R&D systems).

### In vitro angiogenesis assay

The formation of capillary-like tubular structures was assessed in Matrigel-coated multiwell plates essentially as described previously.¹⁶ Freshly isolated VEGFR1+, VEGFR-2+ and Tie2+ cells were seeded at a density of 30 x 10⁴ cells/ml in growth medium in Matrigel-coated multiwell plates, and were incubated at 37°C. The formation of tubular structures was examined after 22 hrs using a Nikon TE300 microscope.

The formation of capillary tube-like structures by freshly isolated CD14+/VEGFR-2+ and CD14+/VEGFR-2- cells was assessed in a solubilized basement membrane preparation extracted from the Engelbreth-Holm-Swarm mouse sarcoma, frequently used for the evaluation of *in vitro* angiogenesis. Twenty-four well plates were coated with 200 µl of Matrigel (pregelled for 30 minutes at 37 °C in 5% CO₂) and the cells were seeded on the polymerized matrix at a density of 5x10⁴ cells/well. Resulting tube-like structures were examined using a phase-contrast light microscope after cells were cultivated for one week at 37°C in 5% CO₂. HAECs were used as positive control cells.

### In vitro cell invasion assay

Cell invasion through reconstituted basement membrane Matrigel was assayed by a method reported previously.¹⁷ Migrated cells were counted in five different fields under a light microscope at x100 magnification. Each experiment was performed in triplicate wells and repeated four times.

### Lentiviral transduction of FACS sorted CD14+/VEGFR-2+ and CD14+/VEGFR-2- cells

The recombinant lentiviral vector was produced using a three-plasmid expression system as described earlier.¹⁷ For transduction, FACS sorted cells were transferred to 10 ml tubes with 1 ml culture medium and incubated with concentrated pHR'EF1-GFPSIN virions at a MOI of 0.1 at 37 °C in a 5% CO₂ atmosphere overnight. After virus incubation, cells were washed once in PBS and directly injected in the denuded femoral artery of nude mice (see below). To detect GFP expression, following the same transduction procedure, transduced cells were cultured in six-well plate and observed on day six post-transduction by fluorescent microscopy.

### Mice

Balloon injury of the right femoral artery was performed as follows: nude C57 black mice (Taconic M&B, Denmark) with body weight of 20-25 g were anesthetized with isofluorane, and the right femoral artery was exposed to the level of bifurcation through a trans-abdominal incision. Microclamps (S&T, Switzerland) were placed on lower aorta, left iliac artery and the distal part of the right femoral artery. A 2F Fogarty balloon catheter (Baxter) was introduced into the right femoral artery, inflated, and withdrawn three times with rotation. Inflation was performed via the cannula and 1ml of Ringer solution with free outflow through the microincision. Cells (5 x 10⁵ cells per animal) in 100 µl MCDB medium (CD14+/VEGFR-2+, *n*=10; CD14+/VEGFR-2-, n=10), were instilled through the same cannula and incubated in the freshly injured arterial bed for 15-20 min while control-transplanted mice received only culture medium (mice, n=6). After incubation, unbound cells were aspirated, the catheter was removed, and the micro-incision was sutured with 11-0 nylon (S&T, Switzerland) interrupted suture. By removing the micro-clamps the blood flow was restored. All animal procedures were performed in accordance with institutional guidelines and conformed to the guide for the Care and Use of Laboratory Animals at Huddinge University hospital in Sweden. Animals were sacrificed at 2 and 4 weeks after human cell transplantation and the right femoral artery was harvested for histopathological examinations. Vessels were embedded in O.C.T. and frozen in liquid nitrogen.

To demonstrate activation of DNA synthesis in the human VEGFR-2+ and Tie-2+ cells *in vivo*, the thymidine analogue BrdU (Sigma, Sweden) was given as single i.p injections 100mg/kg body weight 12 and 24 hrs prior to termination.

### Immunohistochemistry

To localize human cells in the mouse artery, 5-µm cryosections were stained with an anti-GFP antibody (1:50). The immunoperoxidase procedure was carried out using Vectastain Elite ABC kit (ImmunKemi, Stockholm, Sweden) as described by the manufacturers. The ACE (gives red staining) substrate kit was used as color developer and counter-stained with hematoxylin. In addition, a mouse anti-human nuclei monoclonal antibody (Chemicon, CA, USA) was used to detect human cells in the mouse artery by immunofluorescence.¹⁸ The phenotype of the transplanted human cells was detected by double staining with antibodies to human nuclei (1:50), and anti-VE-Cadherin (1:100). This was followed by staining with secondary anti-mouse subclass-specific FITC or Texas red conjugated antibodies (1:500). Endothelialization was calculated as the percentage of the surface covered by human ACE positive cells and the total luminal surface. For histomorphology (six animals per group, 6 sections/femoral artery) crosssections of the artery were stained with hematoxylin/eosin and examined for vessel diameter, media area, and intima to media area ratio.

### Reverse transcriptase-polymerase chain reaction (RT-PCR)

Total RNA was extracted from three human-mouse chimeric femoral arteries, one mouse receiving CD14+/VEGFR-2- cells and one sham transplanted mouse at 4 weeks after transplantation, using the Micro-FastTrack RNA isolation kit (Invitrogen, Groningen, The Netherlands). Human specific primers were used to detect human CD31 and e-NOS in the human cell-transplanted mice arteries. Freshly isolated CD14+/VEGFR-2+ and CD14+/VEGFR-2- cells were also tested for expression of e-NOS. ABL was used as an endogenous reference gene. Primer sets were commercially synthesized by CyberGene (Huddinge, Sweden). Primer sequences were
"CD31-F": 5'-CCA CTG CAG AGT ACC AGG TGT TGG-3',
"CD31-R": 5'-ATC GAG AAG GAG CGT TTC T-3', expected product size (bp):248.
"e-NOS-F": 5'-CTG TAT GGC TCC GAG ACC-3'
"e-NOS-R": 5'-GCT GTT GAA GCG GAT CTT ATA AC-3', expected product size (bp):279
"ABL-F": 5'-CGG CTC TCG GAG GAG ACG ATG A-3',
"ABL-R": 5'-CCC AAC CTT TTC GTT GCA CTG T-3', expected product size (bp):385.

The PCR reaction was carried out as described earlier. 19

### Statistical analysis

Where applicable, results are presented as mean ±SD. Unpaired t test was used for comparisons between EPC-transplanted and sham (medium only) treated groups. p<0.05 was considered to be significant. Mann-Whitney U test was used for comparisons between the groups. p<0.05 was considered to be significant.

### EXAMPLE 2: VEGFR-1+, VEGFR-2+ AND TIE-2+ CELLS FROM PERIPHERAL BLOOD BELONG TO THE MONOCYTE/MACROPHAGE LINEAGE

It was found that VEGFR-1+, VEGFR-2+ and Tie-2+ cells constitute approximately 3± 0.2%, 0.8±0.5% and 2±0.3% of the total population of mononuclear cells in blood respectively. The phenotypic analysis of the three populations demonstrated a mixture of cell types belonging mainly to the monocyte/macrophage cell lineage (Table 1). Freshly isolated VEGFR-1+ and Tie-2+ cells did not express the endothelial cell marker VE-Cadherin (CD144). However, a small percentage of the freshly isolated VEGFR-2+ cells expressed this marker. In addition, all three populations expressed the CD45 marker indicating the hematopoietic origin of these cells. VEGFR-1+, VEGFR-2+ and Tie-2+ cells stained positive for their respective markers (Fig. 1A-C). Electron microscopic studies of freshly isolated VEGFR-1+, VEGFR-2+ and Tie-2 + cells further supported the findings of the phenotypic analysis. All three populations contained a mixture of cells including mononuclear monocytes, macrophages, lymphocytes and polymorphonuclear cells (Fig. 1D-F).

**Table 1 Phenotypic characteristics of freshly isolated VEGFR-1+, VEGFR-2+ and Tie-2+ cells from peripheral blood**

| **Antibodies to** | **VEGFR-1+ cells** | **VEGFR-2+ cells** | **Tie-2+ cells** | **HUVECs %** |
|---|---|---|---|---|
| | **(n=7) %** | **(n=7) %** | **(n=7) %** | **(cell line)** |
| **Monocyte/macrophage markers** | | | | |
| CD15 | 5.5 ± 0.4 | 5 ± 0.3 | 13 ± 0.2 | 0 |
| CD64 | 60 ± 0.03 | 15 ± 0.01 | 80 ± 0.05 | 0 |
| CD14 | 93 ± 0.02 | 80 ± 0.04 | 75 ± 0.25 | 0 |
| CD11b | 97 ± 0.02 | 81 ± 0.1 | 90 ± 0.01 | 0 |
| CD11c | 72 ± 0.2 | 54 ± 0.4 | 65 ± 0.1 | 0 |
| CD68 | 0 | 0.4 ± 0.05 | 2 ± 0.5 | 0 |
| CD83 | 0.49 ± 0.05 | 3.7 ± 0.05 | 0.07 ± 0.01 | 0 |

| | **Stem/progenitor/mature endothelial cell markers** | | | |
|---|---|---|---|---|
| CD34 | 0.05 ± 0.1 | 1.0 ± 0.02 | 0.19 ± 0.1 | 5 |
| CD123 | 4 ± 0.03 | 2 ± 0.02 | 5 ± 0.03 | 0 |
| AC133 | 0.21 ± 0.02 | 0.2 ± 0.01 | 0.8 ± 0.01 | 0 |
| CD90 | 0.7 ± 0.3 | 1.3 ± 0.1 | 2 ± 0.3 | 0 |
| AcLDL | 100 | 100 | 100 | 100 |
| CD31 | 75 ± 0.04 | 70 ± 0.2 | 77 ± 0.03 | 100 |
| CD105 | 100 | 100 | 100 | 100 |
| VEGFR-2 | 52± 0.4 | 92 ± 0.03 | 6± 0.4 | 12 |
| VEGFR-1 | 95 ± 0.03 | 5 ± 0.03 | 2.7 ± 0.01 | 7 |
| Tie-2 | 4.3 ± 0.03 | 55 ± 0.2 | 94 ± 0.03 | 12 |
| CD144 | 0 | 6 ± 0.03 | 2± 0.25 | 100 |
| CD141 | 1 ± 0.05 | 6 ± 0.2 | 5± 0.13 | 100 |
| CD142 | 4±0.06 | 3±0.02 | 4 ± 0.02 | 100* |
| HLA class I | 90 ± 0.03 | 83 ± 0.03 | 80 ± 0.05 | 100 |
| HLA-DR | 20 ± 0.03 | 23 ± 0.03 | 29 ± 0.01 | 100* |
| Ulex europaes | 100 | 100 | 100 | 100 |

| **Control markers** | | | | |
|---|---|---|---|---|
| a-actin | 1 ± 0.03 | 1.0 ± 0.01 | 1.0 ± 0.07 | 0 |
| Fibroblasts | 6.6 ± 0.01 | 38.45 ± 0.01 | 0.1 ± 0.05 | 0 |
| CD45 | 100 | 94 ± 0.03 | 63 ± 0.03 | 0 |
| CD3 | 0 | 0 | 0 | 0 |
| CD56+16 | 0 | 0 | 0 | 0 |

### EXAMPLE 3: CULTIVATED VEGFR-2+ AND TIE-2+ CELLS EXHIBIT EXCLUSIVELY ENDOTHELIAL CELLS MARKERS

Cultivation of these cells on fibronectin-coated culture wells showed two populations of adherent (80%) and non-adherent (20%) cells. The non-adherent cells were discarded after one week and could not be further cultivated under the conditions described for endothelial cells. After two weeks in culture, the majority of the adherent cells in the VEGFR-2+ and Tie2+ cultures expressed the endothelial cell markers CD144, CD141 and upon activation expressed CD106 and CD142 (Table 2). Further analysis using electron microscopy confirmed the endothelial morphology of these cells, demonstrating the presence of fenestrae, weibel-palade bodies and tight junctions typical for endothelial cells (Fig. 1G-I). On the other hand, only a small fraction of VEGFR-1+ cells remained adherent after two weeks and sufficient cells were not obtained for further analysis. Neither VEGFR-2+ nor Tie-2+ cells proliferated in culture. However, these populations could be kept viable in culture without proliferation for >4 weeks.

**Table 2 Phenotypic characteristics of VEGFR-2+ and Tie-2+ cells after two weeks in culture**

| **Antibodies to** | **VEGFR-2+ cells** | **Tie-2+ cells** |
|---|---|---|
| | **(n=7) %** | **(n=7) %** |
| CD 141 | 95±4.5 | 90±3.1 |
| *CD 142 | 98±1.9 | 96±4.2 |
| CD 144 | 95±3.8 | 91±3.8 |
| CD14 | 0 | 0 |
| CD31 | 92±7.2 | 90±6.8 |
| *CD 106 | 100 | 100 |
| *CD 62E | 20±4.3 | 20±6.2 |
| CD11b | 0 | 0 |
| CD15 | 0 | 0 |
| CD68 | 0 | 0 |
| Fibroblasts | 0.07±3.2 | 0.08±1.5 |
| Alpha-actin | 0.5±0.8 | 0.4±0.2 |

### EXAMPLE 5: VEGFR-2+ AND TIE-2+ CELLS HAVE MIGRATORY AND INVASIVE CAPACITY AND FORM TUBULE-LIKE STRUCTURES

Freshly isolated VEGFR-2+ and Tie-2+ but not VEGFR-1+ cells migrated towards VEGF and angiopoietin-2 (Fig. 2A & B). However, 60% VEGFR-1+, 70% VEGFR-2+ and 65% Tie2+ cells added to matrigel migrated to the lower chamber in the invasion assay indicating that all three cell types had invasive capacity (Fig. 2C). In matrigel, these freshly isolated cells showed that only VEGFR-2+ and Tie-2+ cells could form tubule-like structures but VEGFR1+ cells did not have the ability to do so (Fig. 2D-G). HUVEC were used as controls. Our *in vitro* studies showed that the VEGFR-2+ and Tie-2+ cells phenotypically and morphologically resembled endothelial cells, while VEGFR-1+ cells did not exhibit endothelial-like characteristics. Therefore the *in vivo* functional capacity of VEGFR-2+ and Tie-2+ cells to take part in reendothelialization of balloon-injured arteries was further analysed.

### EXAMPLE 6: VEGFR-2+ AND TIE-2+ CELLS SIGNIFICANTLY CONTRIBUTED TO EFFICIENT REPOPULATION OF DENUDED MOUSE FEMORAL ARTERIES AND PROLIFERATED IN VIVO

An experimental angioplasty procedure was performed in the femoral artery of nude mice. Immediately following the intervention, VEGFR-2+ or Tie-2+ cells were locally infused into the freshly injured arterial bed. Transplanted vessels showed no sign of thrombosis. The specificity of the human-nuclei antibody was first determined by using a normal human and mouse artery. Human cells stained black in color (Fig. 3A). Control mouse artery did not stain with the human nuclei antibody (Fig. 3B). At two weeks after transplantation human VEGFR2+ cells were detected in the luminal surface of the seeded balloon-injured femoral artery ((DAB-Ni-positive Fig. 3C). At 4 weeks post-transplantation a high degree of repopulation was seen and approximately 75±8% of the lesion was covered with human VEGFR-2+cells. Similarly, Tie-2+ cells were detected at 2 and 4 weeks after transplantation (Fig. 3D). Animals transplanted with Tie-2+ cells at 4 weeks post-transplantation showed a high degree of repopulation and approximately 70±5% of the lesion was covered with human Tie-2+ cells.

The presence of human cells in the mouse artery was once again confirmed in the immunofluorescence studies (Fig. 3E-F). EC phenotype *in vivo* was confirmed by staining with antibodies to von Willebrand factor, and VE-Cadherin (CD144 )(Fig. 4A & B). Each section was double-stained with the antihuman nuclear antibody to differentiate human cells from host cells (yellow staining indicating double positive cells). Interestingly, transplanted human cells were found not only in the luminal surface of the seeded arteries but also in the interstitial media. When labeled with an antibody to α-actin, cells in the media stained positive indicating the presence of smooth muscle cells (Fig. 4G-I). Thus, both VEGFR-2+ and Tie-2+ cells differentiated *in vivo* not only into endothelial cells but also smooth muscle cells. Neither VEGFR-2+ nor Tie-2+ cells proliferated *in vitro* (data not shown), but to obtain evidence for proliferation in the transplanted VEGFR-2+ and Tie-2+ cells, BrdU incorporation studies were performed in eight animals (VEGFR2+ n=4, Tie-2+ n=4). Analysis of tissue at 7 days showed extensive BrdU incorporation in transplanted human cells. Since both the anti-BrdU and the human-nuclei antibodies stain DNA, double staining of the same tissue section was not possible. Therefore, consecutive sections were stained with antiBrdU (brown) and the anti-human nuclei antibody (black) to show proliferation in the transplanted cells (Fig. 5A-F). These findings show that VEGFR-2+ and Tie-2+ cells not only repopulate injured arteries but also proliferate *in vivo.*

### EXAMPLE 7: RE-ENDOTHELIALIZATION WITH ENDOTHELIAL PROGENITOR CELLS DID NOT RESULT IN NEOINTIMA PROLIFERATION FOLLOWING BALLOON INJURY OF MICE FEMORAL ARTERIES

Cross-sections of injured arterial segments were examined at 2 and 4 weeks post transplantation for histomorphological changes. The balloon-injury procedure in the mouse femoral artery resulted in dilation of the lesioned arterial segment so that the vessel diameter was significantly increased from 0.362±0.060 mm in uninjured control arteries to 0.601±0.036 mm in injured vessels that were incubated with media alone. This widening was not found in femoral arteries of mice that had received transplantation of human cells (Fig. 5G). A reduction in cross-sectional media area from 0.020±0.004 to 0.010±0.009 mm² was observed in injured arteries. Thinning out of media area was not seen in VEGFR-2 and Tie-2 cells transplanted arteries Fig. 5H. Reendothelialization mediated by EPC seeding did not result in neointima formation in femoral arteries at 2, 4 or 6 weeks post injury. In fact, intimal to media area ratio was not significantly higher in VEGFR-2 and Tie-2 transplanted vessel segments (I/M ratio: normal uninjured artery 0.01±0.01, media control 0.02±0.01, VEGFR-2+ 0.02±0.01; Tie-2+ 0.02±0.01, p=ns).

### EXAMPLE 8: CHARACTERIZATION OF CD14+/VEGFR-2+ AND CD14+/VEGFR-2-

It was found that CD14+/VEGFR-2+ cells constitute approximately 2± 0.5%, (Fig. 1A) of the total population of monocytes and 0.08 ± 0.04% of PBMCs in blood. Immunocytochemical analysis demonstrated that the freshly isolated CD14+/VEGFR-2- cell fraction did not express e-NOS (Fig. 6B). However, freshly isolated CD14+/VEGFR-2+ cells expressed e-NOS, von Willebrand Factor and thrombomodulin (Fig. 6C) but not VE-Cadherin. Expression of the VEGFR-2 receptor on CD14+/VEGFR-2+ cells was further supported by the fact that, lysates of this population when immunoblotted with anti-VEGFR-2 antibodies gave the expected band of approximately 200 kDa which was absent or faintly expressed in the CD14+/VEGFR-2- cell population (Fig. 6D). Furthermore the e-NOS mRNA was strongly expressed in CD14+/VEGFR-2+ cells, while in the VEGFR-2 negative fraction only a faint e-NOS mRNA band was visible (Fig. 6E). We cannot rule out the fact that this faint band could be due to a small number of contaminating VEGFR-2+ cells in the CD14+/VEGFR-2- cell population. The phenotypic markers expressed by the CD14+/VEGFR-2- population is shown in Table 3. Both populations expressed the CD45 marker indicating the hematopoietic origin of these cells. Electron microscopic analysis of CD14+/VEGFR-2+ cells cultured on Transwell tissue culture inserts demonstrated formation of a basement membrane, the presence of Weibel Palade bodies and tight junctions characteristic of endothelial cells (Fig.7A-C), while the negative fraction did not exhibit these features.

CD14+/VEGFR-2+ cells when cultivated on fibronectin-coated culture wells formed colonies after three days in culture. These colonies over the course of the next few days changed morphology from a center of densely packed round cells to seemingly mature flattened, elongated cells. On the other hand, CD14+/VEGFR-2- population remained adherent as slightly rounded cells for two weeks but did not form clusters (Fig. 7D). Neither of the two populations demonstrated extensive proliferative capacity under the culture conditions used in the present study. Cultivated CD14+/VEGFR-2+ cells expressed VE-Cadherin after 1 week in culture (Fig. 7D), while the VEGFR-2- fraction did not express this marker at any time point during cultivation.

**Table 3**

| **Phenotypic characteristics of freshly isolated CD14+/VEGFR-2- cells from peripheral blood** | | |
|---|---|---|
| **Antibodies to** | **CD14+/VEGFR-2-cells** **(n=5) %** | **HAECs %** **(primary cell line)** |
| **Stem/progenitor/mature endothelial cell markers** | | |
| VEGFR-2 | 0.005 ± 0.001 | 10 |
| VEGFR-1 | 81 ± 1.33 | 6 |
| Tie-2 | 23 ± 2.5 | 15 |
| CD34 | 0.01 ± 0.007 | 5 |
| CD123 | 28 ± 2.1 | 0 |
| CD133 | 0.05 ± 0.002 | 0 |
| CD90 | 0 | |
| AcLDL | 100 | 100 |
| CD31 | 20 ± 2.6 | 100 |
| CD144 | 0 | 100 |
| vWF | 0 | 100 |
| Ulex europaeus | 100 | 100 |

| **Monocyte/macrophage markers** | | |
|---|---|---|
| CD14 | 197 ± 2.2 | |
| CD15 | 8 ± 3.2 | 0 |
| CD11b | 92 ± 2.4 | |
| CD11c | 84 ± 2.7 | 0 |
| CD68 | 3 ± 1.5 | 0 |
| CD83 | 2.7 ± 1.4 | 0 |

| **Control markers** | | |
|---|---|---|
| alpha-actin | 0 | 0 |
| Fibroblasts | 0 | 0 |
| CD45 | 100 | 0 |
| CD3 | 0 | 0 |
| CD56 | 0 | 0 |
| CD16 | 2 ± 0.4 | 0 |
| CD19 | 0 | 0 |

### EXAMPLE 9: IN VITRO FUNCTIONAL CAPACITY OF CD14+/VEGFR-2+ AND CD14+/VEGFR-2- PERIPHERAL BLOOD CELLS

Significantly higher numbers of freshly isolated CD14+/VEGFR-2+ cells migrated towards VEGF and MCP-1 as compared with CD14+/VEGFR-2- cells (Fig. 8A) (p=0.009 and p=0.047 respectively). The angiogenic growth factors produced by the two populations did not vary significantly. Both populations produced VEGF, G-CSF and GM-CSF but not SDF-1 or FGF.

However, growth factor levels were higher in the CD14+/VEGFR-2+ population as compared to the CD14+/VEGFR-2- population (Fig. 8B). In matrigel, three-days cultivated cells showed that only CD14+/VEGFR-2+ but not CD14+/VEGFR-2- cells could form tubule-like structures (Fig. 8C). HAECs were used as controls.

Thus, the *in vitro* data indicated that, freshly isolated CD14+/VEGFR-2+ cells demonstrated endothelial-like characteristics as compared to CD14+/VEGFR-2- cells.

### EXAMPLE 10: CD14+/VEGFR-2+ CELLS DIFFERENTIATED TO ENDOTHELIAL CELLS AND EFFICIENTLY REPOPULATED DENUDED MOUSE FEMORAL ARTERIES

Exposure of the two cell populations to lentiviral particles expressing GFP led consistently to transduction efficiencies of >70%. Screening of artery sections for human cells demonstrated that due to high auto-fluorescence, visualisation of GFP+ cells was difficult. Therefore an enzyme-based immunohistochemical staining was performed using anti-GFP antibody to detect GFP+ cells.

Using this antibody no GFP-positive areas in sham-transplanted and CD14+/VEGFR-2-transplanted mice were found (Fig. 9A-C). However, coverage of the intimal surface of arteries with GFP-positive cells (red) was seen already at 2 weeks after transplantation in mice receiving CD14+/VEGFR-2+ cells (Fig. 9D). The GFP-positive area was significantly increased in vessels harvested 4 weeks after seeding (Fig. 9E & F). In addition, human cells were also identified by an anti-human nuclei antibody using immunofluorescence. The specificity of the human-nuclei antibody was first determined by using a normal human and mouse artery. Human cell-nuclei stained red in color (Fig. 10A). Control mouse artery did not stain with the human nuclei antibody (Fig. 5B). At 4 weeks post-transplantation a high degree of repopulation was seen and approximately 70±8% of the lesion was covered with human cells (red) in mice receiving CD14+/VEGFR-2+ human cells (Fig. 10C) but not in mice transplanted with CD14+NEGFR-2-cells (Fig.10D). Animals transplanted with CD14+/VEGFR-2- cells did not demonstrate the presence of these cells in the denuded arteries, spleen, lungs or in circulation at 2 or 4 weeks after transplantation (data not shown). Endothelial cell phenotype *in vivo* was confirmed by staining with antibodies to VE-Cadherin (green) and each section was double-stained with the anti-human nuclei antibody to detect human cells (red). The double stained cells are stained yellow (Fig. 10E).

Cross-sections of injured arterial segments were examined at 2 and 4 weeks post transplantation for histomorphological changes. The balloon-injury procedure in the mouse femoral artery resulted in dilation of the lesioned arterial segment so that the inner vessel diameter was significantly increased from 0.301±0.041 mm in uninjured control arteries to 0.601±0.043 and 0.631±0.028 mm in injured vessels that were incubated with media alone or CD14+/ VEGFR-2-respectively (p<0.001). This widening was not found in femoral arteries of mice 0.404±0.08 mm that were transplanted with CD14+/VEGFR-2+ cells. A reduction in cross-sectional media area from 0.028±0.010 to 0.012±0.007 mm2 was observed in injured arteries. Thinning out of media area was not seen in CD14+/VEGFR-2+ cell transplanted arteries 0.033±0.013. Re-endothelialization mediated by these cells did not result in neointima formation in femoral arteries at 2 or 4 weeks post injury. The intimal to media area ratio was not significantly higher in CD14+/VEGFR-2+ transplanted vessel segments (I/M ratio: normal uninjured artery 0.03±0.009, media control 0.02±0.008, CD14+/VEGFR-2- 0.02±0.009; CD14+/VEGFR-2+ 0.04±0.02, p=ns).

### EXAMPLE 11: TRANSCRIPTION OF HUMAN ENDOTHELIAL-SPECIFIC GENES IN MICE TRANSPLANTED WITH CD14+/VEGFR-2+ CELLS

The engraftment of the transplanted cells was conformed by determining the expression of human genes in transplanted mice. Femoral arteries were analyzed of the mice sacrificed one month after transplantation with human cells by RT-PCR using primers specific for the human endothelial-specific gene CD31 and e-NOS. The CD31 and e-NOS primers were species specific for human as they did not amplify the respective mouse gene (Fig. 11A). These results were obtained with arteries from mice receiving CD14+/VEGFR2+ cells, but not in those transplanted with CD14+/VEGFR-2- cells. These data demonstrate that the transplanted human CD14+/VEGFR-2+ cells engraft the damaged mouse artery.

## Claims

1. A method of producing a population of human progenitor cells which are capable of cultivation in culture and are capable of proliferating and differentiating *in vivo* into endothelial cells or smooth muscle cells, the method comprising selecting from a population of human peripheral blood derived cells for cells that are CD144⁻, CD45⁺ and Tie-2⁺ or VEGFR-2⁺,
and wherein said selecting comprises positive selection for one or more antigens characteristic of EPCs and wherein at least one of said antigens characteristic of EPCs is VEGFR-2 or Tie-2.

2. A method of producing a population of human progenitor cells which are capable of cultivation in culture and are capable of proliferating and differentiating *in vivo* into endothelial cells, the method comprising selecting from a population of human peripheral blood derived cells for cells that are CD144⁻, CD45⁺, CD14+ and VEGFR-2⁺,
and wherein said selecting comprises positive selection for one or more antigens characteristic of EPCs and wherein at least one of said antigens characteristic of EPCs is VEGFR-2.

3. A method according to claim 1 or 2 wherein the method comprises a mixture of both positive and negative selection in either order.

4. A method according to any one of the preceding claims wherein the method further comprises cultivating the cells *in vitro.*

5. A method according to any one of the preceding claims wherein the method further comprises cryopreserving the cells.

6. A method according to claim 4 wherein the cells are viable for at least 2 weeks in culture.

7. A method according to claim 4 wherein the cells are viable for at least 4 weeks in culture.

8. A method according to claim 4 wherein the cells are cultivated as an adhesion culture.

9. A method of manufacturing a medicament for treating a subject suffering from a vascular disorder / disease, vascular tissue damage or a coronary disorder, wherein the method of manufacture comprises producing a population of human progenitor cells according to any one of claims 1 to 8 and formulating the population as a medicament for: (i) transplanting progenitor cells into a subject; (ii) repopulating an injured blood vessel in a subject; or (iii) endothelizing a vascular graft in a subject, which vascular graft is contacted in vivo with the medicament.

10. The method of claim 9(ii), wherein said medicament is for administration directly to the blood vessel.

11. The method of claim 9(ii), wherein said medicament is for administration directly to an arterial bed.

12. The method of claim 9(ii), wherein said blood vessel is a vein or an artery.

13. The method of claim 9(iii), wherein said graft is an artificial vascular graft.

14. The method of claim 9(iii), wherein said graft is an allograft.

15. The method of claim 9(iii), wherein said graft is an isograft.

16. A method of endothelizing a vascular graft, comprising producing a population of human progenitor cells according to any one of claims 1 to 8 and contacting *in vitro* or *ex vivo* said vascular graft with a composition comprising cells of said population.

17. The method of claim 9(iii) or the method of claim 16, wherein said vascular graft is a vein or an artery.

## Patentansprüche

1. Verfahren zur Herstellung einer Population menschlicher Vorläuferzellen, die in Kultur gezüchtet werden können und in der Lage sind, in vivo zu proliferieren und zu differenzieren in Endothelzellen oder glatte Muskelzellen, wobei das Verfahren ein Selektieren aus einer Population von aus menschlichem Peripherblut stammenden Zellen hinsichtlich Zellen, die CD144⁻, CD45⁺ und Tie-2⁺ oder VEGFR-2⁺ sind, aufweist,
und wobei das Selektieren eine positive Selektion hinsichtlich eines oder mehrerer Antigene, die charakteristisch für EPCs (Endothel-Vorläuferzellen) sind, aufweist, und wobei mindestens eines der für EPCs charakteristischen Antigene VEGFR-2 oder Tie-2 ist.

2. Verfahren zur Herstellung einer Population menschlicher Vorläuferzellen, die in Kultur gezüchtet werden können und in der Lage sind, in vivo zu proliferieren und zu differenzieren in Endothelzellen, wobei das Verfahren ein Selektieren aus einer Population von aus menschlichem Peripherblut stammenden Zellen hinsichtlich Zellen, die CD144⁻, CD45⁺, C14⁺ und VEGFR-2⁺ sind, aufweist,
und wobei das Selektieren eine positive Selektion hinsichtlich eines oder mehrerer Antigene, die charakteristisch für EPCs sind, aufweist, und wobei mindestens eines der für EPCs charakteristischen Antigene VEGFR-2 ist.

3. Verfahren nach Anspruch 1 oder 2, wobei das Verfahren eine Mischung aus sowohl einer positiven als auch einer negativen Selektion in der einen oder der anderen Reihenfolge aufweist.

4. Verfahren nach einem der vorangehenden Ansprüche, wobei das Verfahren außerdem ein Züchten der Zellen in vitro aufweist.

5. Verfahren nach einem der vorangehenden Ansprüche, wobei das Verfahren außerdem ein Kryokonservieren der Zellen aufweist.

6. Verfahren nach Anspruch 4, wobei die Zellen in Kultur mindestens zwei Wochen lang lebensfähig sind.

7. Verfahren nach Anspruch 4, wobei die Zellen in Kultur mindestens vier Wochen lang lebensfähig sind.

8. Verfahren nach Anspruch 4, wobei die Zellen als eine Adhäsionskultur gezüchtet werden.

9. Verfahren zur Herstellung eines Arzneimittels zur Behandlung eines Individuums, das unter einer Gefäß-Störung/Krankheit, einer Gefäßgewebeschädigung oder einer Herzkranzgefäßerkrankung leidet, wobei das Herstellungsverfahren die Herstellung einer Population menschlicher Vorläuferzellen nach einem der Ansprüche 1 bis 8 und die Formulierung der Population als ein Arzneimittel aufweist zum: (i) Transplantieren von Vorläuferzellen in ein Individuum; (ii) Wiederbesiedeln eines verletzten Blutgefäßes in einem Individuum; oder (iii) Endothelisieren eines Gefäßtransplantats in einem Individuum, wobei das Gefäßtransplantat in vivo mit dem Arzneimittel in Kontakt gebracht wird.

10. Verfahren nach Anspruch 9 (ii), wobei das Arzneimittel zur Verabreichung unmittelbar zu dem Blutgefäß ist.

11. Verfahren nach Anspruch 9 (ii), wobei das Arzneimittel zur Verabreichung unmittelbar zu einem Arterienbett ist.

12. Verfahren nach Anspruch 9 (ii), wobei das Blutgefäß eine Vene oder eine Arterie ist.

13. Verfahren nach Anspruch 9 (iii), wobei das Transplantat ein künstliches Gefäßtransplantat ist.

14. Verfahren nach Anspruch 9 (iii), wobei das Transplantat ein Allotransplantat ist.

15. Verfahren nach Anspruch 9 (iii), wobei das Transplantat ein Isotransplantat ist.

16. Verfahren zum Endothelisieren eines Gefäßtransplantats, aufweisend die Herstellung einer Population menschlicher Vorläuferzellen nach einem der Ansprüche 1 bis 8 und das in Kontakt bringen des Gefäßtransplantats in vitro oder ex vivo mit einer Zusammensetzung, die Zellen der Population aufweist.

17. Verfahren nach Anspruch 9 (iii) oder Verfahren nach Anspruch 16, wobei das Gefäßtransplantat eine Vene oder eine Arterie ist.

## Revendications

1. Procédé de production d'une population de cellules progénitrices humaines qui sont capables d'être cultivées en culture et sont capables de proliférer et de se différencier in vivo en cellules endothéliales ou en cellules de muscles lisses, le procédé comprenant la sélection, à partir d'une population de cellules issues de sang périphérique humain, de cellules qui sont CD144⁻, CD45⁺ et Tie-2⁺ ou VEGFR-2⁺,
et dans lequel ladite sélection comprend une sélection positive pour un ou plusieurs antigènes caractéristiques des EPC et dans lequel au moins l'un desdits antigènes caractéristiques des EPC est VEGFR-2 ou Tie-2.

2. Procédé de production d'une population de cellules progénitrices humaines qui sont capables d'être cultivées en culture et sont capables de proliférer et de se différencier in vivo en cellules endothéliales, le procédé comprenant la sélection, à partir d'une population de cellules issues de sang périphérique humain, de cellules qui sont CD144⁻, CD45⁺, CD14⁺ et CEGFR-2⁺,
et dans lequel ladite sélection comprend une sélection positive pour un ou plusieurs antigènes caractéristiques des EPC et dans lequel au moins l'un desdits antigènes caractéristiques des EPC est VEGFR-2.

3. Procédé selon la revendication 1 ou 2, lequel procédé comprend un mélange de sélections tant positive que négative, dans un ordre quelconque.

4. Procédé selon l'une quelconque des revendications précédentes, lequel procédé comprend en outre la culture des cellules in vitro.

5. Procédé selon l'une quelconque des revendications précédentes, lequel procédé comprend en outre une cryoconservation des cellules.

6. Procédé selon la revendication 4, dans lequel les cellules sont viables pendant au moins 2 semaines en culture.

7. Procédé selon la revendication 4, dans lequel les cellules sont viables pendant au moins 4 semaines en culture.

8. Procédé selon la revendication 4, dans lequel les cellules sont cultivées sous la forme d'une culture en adhésion.

9. Procédé de fabrication d'un médicament pour le traitement d'un sujet souffrant d'une maladie/d'un trouble vasculaire, d'un endommagement d'un tissu vasculaire ou d'un trouble coronarien, lequel procédé de fabrication comprend la production d'une population de cellules progénitrices humaines selon l'une quelconque des revendications 1 à 8 et la formulation de la population sous la forme d'un médicament pour : (i) transplantation de cellules progénitrices dans un sujet ; (ii) repopulation d'un vaisseau sanguin lésé chez un sujet ; ou (iii) endothélialisation d'un greffon vasculaire chez un sujet, lequel greffon vasculaire est mis en contact in vivo avec le médicament.

10. Procédé selon la revendication 9(ii), dans lequel ledit médicament est destiné à être administré directement au vaisseau sanguin.

11. Procédé selon la revendication 9(ii), dans lequel ledit médicament est destiné à être administré directement à un lit artériel.

12. Procédé selon la revendication 9(ii), dans lequel ledit vaisseau sanguin est une veine ou une artère.

13. Procédé selon la revendication 9(iii), dans lequel ledit greffon est un greffon vasculaire artificiel.

14. Procédé selon la revendication 9(iii), dans lequel ledit greffon est un allogreffon.

15. Procédé selon la revendication 9(iii), dans lequel ledit greffon est un isogreffon.

16. Procédé d'endothélialisation d'un greffon vasculaire, comprenant la production d'une population de cellules progénitrices humaines selon l'une quelconque des revendications 1 à 8 et la mise en contact, in vitro ou ex vivo, dudit greffon vasculaire avec une composition comprenant des cellules de ladite population.

17. Procédé selon la revendication 9(iii) ou procédé selon la revendication 16, dans lequel ledit greffon vasculaire est une veine ou une artère.
